# EUROPEAN PATENT APPLICATION

(11) **EP 3 747 904 A1**
(43) Date of publication of application: **09.12.2020**
(21) Application number: 19177859.6
(22) Date of filing: 03.06.2019
(51) Int. Cl.: C07K 16/28, A61K 35/17, C07K 14/725, C07K 14/705, C12N 5/0783, A61P 37/00

(54) **CD25-SPECIFIC CHIMERIC ANTIGEN RECEPTORS AND THEIR USES**

(71) Applicant: Universität Regensburg, 93053 Regensburg (DE); Universität zu Köln, 50937 Köln (DE)
(72) Inventor: ABKEN, Hinrich, 94333 Geiselhöring-Hainsbach (DE); EHLING, Manuel, 48607 Ochtrup (DE); HOMBACH, Andreas, 50321 Brühl (DE)
(74) Representative: Engelhard, Markus

(57) **Abstract**

The present invention relates to proteins which comprise (i) a CD25-specific binding domain, (ii) a linker domain, connecting domain (i) and domain (iii), (iii) a transmembrane domain, and (iv) a signalling domain. The present invention furthermore relates to nucleic acids encoding the proteins, expression constructs for expressing the protein in a host cell and host cells. The present invention further relates to pharmaceutical compositions comprising said protein(s), nucleic acid(s), expression construct(s) or host cell(s). The proteins of the invention are CD25-specific chimeric antigen receptors that are suitable for generating CD25-specific immune cells, which can be used e.g. in the treatment of inflammation.

SEQUENCE LISTING

<110>  Universität Regensburg

<120>  CD25-specific chimeric antigen receptors and their uses

<130>  U30921EP

<160>  14

<170>  PatentIn version 3.5

<210>  1
<211>  239
<212>  PRT
<213>  Artificial Sequence

<220>
<223>  anti-CD25 scFv

<400>  1

Gln Val Lys Leu Gln Gln Ser Gly Thr Val Leu Ala Arg Pro Gly Ala
1               5                   10                  15

Ser Val Lys Met Ser Cys Lys Ala Ser Gly Tyr Arg Phe Thr Asn Tyr
            20                  25                  30

Trp Met His Trp Val Lys Gln Arg Pro Gly Gln Gly Leu Glu Trp Ile
        35                  40                  45

Gly Val Ile Tyr Pro Gly Asn Ser Asp Thr Ser Tyr Asn Gln Lys Phe
    50                  55                  60

Lys Gly Lys Ala Lys Leu Thr Ala Val Thr Ser Ala Ser Thr Ala Tyr
65                  70                  75                  80

Met Glu Leu Ser Ser Leu Thr Asn Glu Asp Ser Ala Val Tyr Tyr Cys
                85                  90                  95

Thr Arg Glu Gly Glu Gly Ser Asp Tyr Trp Gly Gln Gly Thr Thr Val
            100                 105                 110

Thr Val Ser Ser Gly Gly Gly Gly Ser Gly Gly Gly Gly Ser Gly Gly
        115                 120                 125

Gly Gly Ser Gln Ile Val Leu Thr Gln Ser Pro Ala Thr Met Ala Ala
    130                 135                 140

Ser Pro Gly Glu Lys Ile Thr Ile Thr Cys Ser Ala Ser Ser Ser Ile
145                 150                 155                 160

Ser Ser Asn Tyr Leu His Trp Tyr Gln Gln Lys Pro Gly Phe Ser Pro

165                 170                 175

Lys Leu Leu Ile Tyr Arg Thr Ser Asn Leu Ala Ser Gly Val Pro Ala
            180                 185                 190

Arg Phe Ser Gly Ser Gly Ser Gly Thr Ser Tyr Ser Leu Thr Ile Gly
        195                 200                 205

Thr Met Glu Ala Glu Asp Val Ala Thr Tyr Tyr Cys Gln Gln Gly Ser
    210                 215                 220

Ser Ile Pro Tyr Thr Phe Gly Gly Gly Thr Lys Leu Glu Leu Lys
225                 230                 235

<210>  2
<211>  236
<212>  PRT
<213>  Artificial Sequence

<220>
<223>  linker domain human IgG1 Fc

<400>  2

Asp Pro Ala Glu Pro Lys Ser Pro Asp Lys Thr His Thr Cys Pro Pro
1               5                   10                  15

Cys Pro Ala Pro Glu Leu Leu Gly Gly Pro Ser Val Phe Leu Phe Pro
            20                  25                  30

Pro Lys Pro Lys Asp Thr Leu Met Ile Ser Arg Thr Pro Glu Val Thr
        35                  40                  45

Cys Val Val Val Asp Val Ser His Glu Asp Pro Glu Val Lys Phe Asn
    50                  55                  60

Trp Tyr Val Asp Gly Val Glu Val His Asn Ala Lys Thr Lys Pro Arg
65                  70                  75                  80

Glu Glu Gln Tyr Asn Ser Thr Tyr Arg Val Val Ser Val Leu Thr Val
                85                  90                  95

Leu His Gln Asp Trp Leu Asn Gly Lys Glu Tyr Lys Cys Lys Val Ser
            100                 105                 110

Asn Lys Ala Leu Pro Ala Pro Ile Glu Lys Thr Ile Ser Lys Ala Lys
        115                 120                 125

Gly Gln Pro Arg Glu Pro Gln Val Tyr Thr Leu Pro Pro Ser Arg Asp
    130                 135                 140

Glu Leu Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Lys Leu Thr Val Asp Lys Ser Arg Trp Gln Gln Gly
        195                 200                 205

Asn Val Phe Ser Cys Ser Val Met His Glu Ala Leu His Asn His Tyr
    210                 215                 220

Thr Gln Lys Ser Leu Ser Leu Ser Pro Gly Lys Lys
225                 230                 235

<210>  3
<211>  234
<212>  PRT
<213>  Artificial Sequence

<220>
<223>  linker domain human IgG1 delta Fc

<400>  3

Pro Ala Glu Pro Lys Ser Pro Asp Lys Thr His Thr Cys Pro Pro Cys
1               5                   10                  15

Pro Ala Pro Pro Val Ala Gly Pro Ser Val Phe Leu Phe Pro Pro Lys
            20                  25                  30

Pro Lys Asp Thr Leu Met Ile Ala Arg Thr Pro Glu Val Thr Cys Val
        35                  40                  45

Val Val Asp Val Ser His Glu Asp Pro Glu Val Lys Phe Asn Trp Tyr
    50                  55                  60

Val Asp Gly Val Glu Val His Asn Ala Lys Thr Lys Pro Arg Glu Glu
65                  70                  75                  80

Gln Tyr Asn Ser Thr Tyr Arg Val Val Ser Val Leu Thr Val Leu His
                85                  90                  95

Gln Asp Trp Leu Asn Gly Lys Glu Tyr Lys Cys Lys Val Ser Asn Lys
            100                 105                 110

Ala Leu Pro Ala Pro Ile Glu Lys Thr Ile Ser Lys Ala Lys Gly Gln
        115                 120                 125

Pro Arg Glu Pro Gln Val Tyr Thr Leu Pro Pro Ser Arg Asp Glu Leu
    130                 135                 140

Thr Lys Asn Gln Val Ser Leu Thr Cys Leu Val Lys Gly Phe Tyr Pro
145                 150                 155                 160

Ser Asp Ile Ala Val Glu Trp Glu Ser Asn Gly Gln Pro Glu Asn Asn
                165                 170                 175

Tyr Lys Thr Thr Pro Pro Val Leu Asp Ser Asp Gly Ser Phe Phe Leu
            180                 185                 190

Tyr Ser Lys Leu Thr Val Asp Lys Ser Arg Trp Gln Gln Gly Asn Val
        195                 200                 205

Phe Ser Cys Ser Val Met His Glu Ala Leu His Asn His Tyr Thr Gln
    210                 215                 220

Lys Ser Leu Ser Leu Ser Pro Gly Lys Lys
225                 230

<210>  4
<211>  611
<212>  PRT
<213>  Artificial Sequence

<220>
<223>  CAR # 1035

<400>  4

Gln Val Lys Leu Gln Gln Ser Gly Thr Val Leu Ala Arg Pro Gly Ala
1               5                   10                  15

Ser Val Lys Met Ser Cys Lys Ala Ser Gly Tyr Arg Phe Thr Asn Tyr
            20                  25                  30

Trp Met His Trp Val Lys Gln Arg Pro Gly Gln Gly Leu Glu Trp Ile
        35                  40                  45

Gly Val Ile Tyr Pro Gly Asn Ser Asp Thr Ser Tyr Asn Gln Lys Phe
    50                  55                  60

Lys Gly Lys Ala Lys Leu Thr Ala Val Thr Ser Ala Ser Thr Ala Tyr
65                  70                  75                  80

Met Glu Leu Ser Ser Leu Thr Asn Glu Asp Ser Ala Val Tyr Tyr Cys
                85                  90                  95

Thr Arg Glu Gly Glu Gly Ser Asp Tyr Trp Gly Gln Gly Thr Thr Val
            100                 105                 110

Thr Val Ser Ser Gly Gly Gly Gly Ser Gly Gly Gly Gly Ser Gly Gly
        115                 120                 125

Gly Gly Ser Gln Ile Val Leu Thr Gln Ser Pro Ala Thr Met Ala Ala
    130                 135                 140

Ser Pro Gly Glu Lys Ile Thr Ile Thr Cys Ser Ala Ser Ser Ser Ile
145                 150                 155                 160

Ser Ser Asn Tyr Leu His Trp Tyr Gln Gln Lys Pro Gly Phe Ser Pro
                165                 170                 175

Lys Leu Leu Ile Tyr Arg Thr Ser Asn Leu Ala Ser Gly Val Pro Ala
            180                 185                 190

Arg Phe Ser Gly Ser Gly Ser Gly Thr Ser Tyr Ser Leu Thr Ile Gly
        195                 200                 205

Thr Met Glu Ala Glu Asp Val Ala Thr Tyr Tyr Cys Gln Gln Gly Ser
    210                 215                 220

Ser Ile Pro Tyr Thr Phe Gly Gly Gly Thr Lys Leu Glu Leu Lys Asp
225                 230                 235                 240

Pro Ala Glu Pro Lys Ser Pro Asp Lys Thr His Thr Cys Pro Pro Cys
                245                 250                 255

Pro Ala Pro Glu Leu Leu Gly Gly Pro Ser Val Phe Leu Phe Pro Pro
            260                 265                 270

Lys Pro Lys Asp Thr Leu Met Ile Ser Arg Thr Pro Glu Val Thr Cys
        275                 280                 285

Val Val Val Asp Val Ser His Glu Asp Pro Glu Val Lys Phe Asn Trp
    290                 295                 300

Tyr Val Asp Gly Val Glu Val His Asn Ala Lys Thr Lys Pro Arg Glu
305                 310                 315                 320

Glu Gln Tyr Asn Ser Thr Tyr Arg Val Val Ser Val Leu Thr Val Leu
                325                 330                 335

His Gln Asp Trp Leu Asn Gly Lys Glu Tyr Lys Cys Lys Val Ser Asn
            340                 345                 350

Lys Ala Leu Pro Ala Pro Ile Glu Lys Thr Ile Ser Lys Ala Lys Gly
        355                 360                 365

Gln Pro Arg Glu Pro Gln Val Tyr Thr Leu Pro Pro Ser Arg Asp Glu
    370                 375                 380

Leu Thr Lys Asn Gln Val Ser Leu Thr Cys Leu Val Lys Gly Phe Tyr
385                 390                 395                 400

Pro Ser Asp Ile Ala Val Glu Trp Glu Ser Asn Gly Gln Pro Glu Asn
                405                 410                 415

Asn Tyr Lys Thr Thr Pro Pro Val Leu Asp Ser Asp Gly Ser Phe Phe
            420                 425                 430

Leu Tyr Ser Lys Leu Thr Val Asp Lys Ser Arg Trp Gln Gln Gly Asn
        435                 440                 445

Val Phe Ser Cys Ser Val Met His Glu Ala Leu His Asn His Tyr Thr
    450                 455                 460

Gln Lys Ser Leu Ser Leu Ser Pro Gly Lys Lys Asp Pro Lys Leu Cys
465                 470                 475                 480

Tyr Leu Leu Asp Gly Ile Leu Phe Ile Tyr Gly Val Ile Leu Thr Ala
                485                 490                 495

Leu Phe Leu Arg Val Lys Phe Ser Arg Ser Ala Asp Ala Pro Ala Tyr
            500                 505                 510

Gln Gln Gly Gln Asn Gln Leu Tyr Asn Glu Leu Asn Leu Gly Arg Arg
        515                 520                 525

Glu Glu Tyr Asp Val Leu Asp Lys Arg Arg Gly Arg Asp Pro Glu Met
    530                 535                 540

Gly Gly Lys Pro Arg Arg Lys Asn Pro Gln Glu Gly Leu Tyr Asn Glu
545                 550                 555                 560

Leu Gln Lys Asp Lys Met Ala Glu Ala Tyr Ser Glu Ile Gly Met Lys
                565                 570                 575

Gly Glu Arg Arg Arg Gly Lys Gly His Asp Gly Leu Tyr Gln Gly Leu
            580                 585                 590

Ser Thr Ala Thr Lys Asp Thr Tyr Asp Ala Leu His Met Gln Ala Leu
        595                 600                 605

Pro Pro Arg
    610

<210>  5
<211>  659
<212>  PRT
<213>  Artificial Sequence

<220>
<223>  CAR # 1036

<400>  5

Gln Val Lys Leu Gln Gln Ser Gly Thr Val Leu Ala Arg Pro Gly Ala
1               5                   10                  15

Ser Val Lys Met Ser Cys Lys Ala Ser Gly Tyr Arg Phe Thr Asn Tyr
            20                  25                  30

Trp Met His Trp Val Lys Gln Arg Pro Gly Gln Gly Leu Glu Trp Ile
        35                  40                  45

Gly Val Ile Tyr Pro Gly Asn Ser Asp Thr Ser Tyr Asn Gln Lys Phe
    50                  55                  60

Lys Gly Lys Ala Lys Leu Thr Ala Val Thr Ser Ala Ser Thr Ala Tyr
65                  70                  75                  80

Met Glu Leu Ser Ser Leu Thr Asn Glu Asp Ser Ala Val Tyr Tyr Cys
                85                  90                  95

Thr Arg Glu Gly Glu Gly Ser Asp Tyr Trp Gly Gln Gly Thr Thr Val
            100                 105                 110

Thr Val Ser Ser Gly Gly Gly Gly Ser Gly Gly Gly Gly Ser Gly Gly
        115                 120                 125

Gly Gly Ser Gln Ile Val Leu Thr Gln Ser Pro Ala Thr Met Ala Ala
    130                 135                 140

Ser Pro Gly Glu Lys Ile Thr Ile Thr Cys Ser Ala Ser Ser Ser Ile
145                 150                 155                 160

Ser Ser Asn Tyr Leu His Trp Tyr Gln Gln Lys Pro Gly Phe Ser Pro
                165                 170                 175

Lys Leu Leu Ile Tyr Arg Thr Ser Asn Leu Ala Ser Gly Val Pro Ala
        180                 185                 190

Arg Phe Ser Gly Ser Gly Ser Gly Thr Ser Tyr Ser Leu Thr Ile Gly
    195                 200                 205

Thr Met Glu Ala Glu Asp Val Ala Thr Tyr Tyr Cys Gln Gln Gly Ser
    210                 215                 220

Ser Ile Pro Tyr Thr Phe Gly Gly Gly Thr Lys Leu Glu Leu Lys Asp
225                 230                 235                 240

Pro Ala Glu Pro Lys Ser Pro Asp Lys Thr His Thr Cys Pro Pro Cys
                245                 250                 255

Pro Ala Pro Glu Leu Leu Gly Gly Pro Ser Val Phe Leu Phe Pro Pro
            260                 265                 270

Lys Pro Lys Asp Thr Leu Met Ile Ser Arg Thr Pro Glu Val Thr Cys
        275                 280                 285

Val Val Val Asp Val Ser His Glu Asp Pro Glu Val Lys Phe Asn Trp
    290                 295                 300

Tyr Val Asp Gly Val Glu Val His Asn Ala Lys Thr Lys Pro Arg Glu
305                 310                 315                 320

Glu Gln Tyr Asn Ser Thr Tyr Arg Val Val Ser Val Leu Thr Val Leu
                325                 330                 335

His Gln Asp Trp Leu Asn Gly Lys Glu Tyr Lys Cys Lys Val Ser Asn
            340                 345                 350

Lys Ala Leu Pro Ala Pro Ile Glu Lys Thr Ile Ser Lys Ala Lys Gly
        355                 360                 365

Gln Pro Arg Glu Pro Gln Val Tyr Thr Leu Pro Pro Ser Arg Asp Glu
    370                 375                 380

Leu Thr Lys Asn Gln Val Ser Leu Thr Cys Leu Val Lys Gly Phe Tyr
385                 390                 395                 400

Pro Ser Asp Ile Ala Val Glu Trp Glu Ser Asn Gly Gln Pro Glu Asn
                405                 410                 415

Asn Tyr Lys Thr Thr Pro Pro Val Leu Asp Ser Asp Gly Ser Phe Phe
            420                 425                 430

Leu Tyr Ser Lys Leu Thr Val Asp Lys Ser Arg Trp Gln Gln Gly Asn
        435                 440                 445

Val Phe Ser Cys Ser Val Met His Glu Ala Leu His Asn His Tyr Thr
    450                 455                 460

Gln Lys Ser Leu Ser Leu Ser Pro Gly Lys Lys Asp Pro Lys Phe Trp
465                 470                 475                 480

Val Leu Val Val Val Gly Gly Val Leu Ala Cys Tyr Ser Leu Leu Val
                485                 490                 495

Thr Val Ala Phe Ile Ile Phe Trp Val Arg Ser Lys Arg Ser Arg Leu
            500                 505                 510

Leu His Ser Asp Tyr Met Asn Met Thr Pro Arg Arg Pro Gly Pro Thr
        515                 520                 525

Arg Lys His Tyr Gln Pro Tyr Ala Pro Pro Arg Asp Phe Ala Ala Tyr
    530                 535                 540

Arg Ser Leu Arg Val Lys Phe Ser Arg Ser Ala Asp Ala Pro Ala Tyr
545                 550                 555                 560

Gln Gln Gly Gln Asn Gln Leu Tyr Asn Glu Leu Asn Leu Gly Arg Arg
                565                 570                 575

Glu Glu Tyr Asp Val Leu Asp Lys Arg Arg Gly Arg Asp Pro Glu Met
            580                 585                 590

Gly Gly Lys Pro Arg Arg Lys Asn Pro Gln Glu Gly Leu Tyr Asn Glu
        595                 600                 605

Leu Gln Lys Asp Lys Met Ala Glu Ala Tyr Ser Glu Ile Gly Met Lys
    610                 615                 620

Gly Glu Arg Arg Arg Gly Lys Gly His Asp Gly Leu Tyr Gln Gly Leu
625                 630                 635                 640

Ser Thr Ala Thr Lys Asp Thr Tyr Asp Ala Leu His Met Gln Ala Leu
                645                 650                 655

Pro Pro Arg

<210>  6

<211>  659
<212>  PRT
<213>  Artificial Sequence

<220>
<223>  CAR # 1037

<400>  6

Gln Val Lys Leu Gln Gln Ser Gly Thr Val Leu Ala Arg Pro Gly Ala
1               5                   10                  15

Ser Val Lys Met Ser Cys Lys Ala Ser Gly Tyr Arg Phe Thr Asn Tyr
            20                  25                  30

Trp Met His Trp Val Lys Gln Arg Pro Gly Gln Gly Leu Glu Trp Ile
        35                  40                  45

Gly Val Ile Tyr Pro Gly Asn Ser Asp Thr Ser Tyr Asn Gln Lys Phe
    50                  55                  60

Lys Gly Lys Ala Lys Leu Thr Ala Val Thr Ser Ala Ser Thr Ala Tyr
65                  70                  75                  80

Met Glu Leu Ser Ser Leu Thr Asn Glu Asp Ser Ala Val Tyr Tyr Cys
                85                  90                  95

Thr Arg Glu Gly Glu Gly Ser Asp Tyr Trp Gly Gln Gly Thr Thr Val
            100                 105                 110

Thr Val Ser Ser Gly Gly Gly Gly Ser Gly Gly Gly Gly Ser Gly Gly
        115                 120                 125

Gly Gly Ser Gln Ile Val Leu Thr Gln Ser Pro Ala Thr Met Ala Ala
    130                 135                 140

Ser Pro Gly Glu Lys Ile Thr Ile Thr Cys Ser Ala Ser Ser Ser Ile
145                 150                 155                 160

Ser Ser Asn Tyr Leu His Trp Tyr Gln Gln Lys Pro Gly Phe Ser Pro
                165                 170                 175

Lys Leu Leu Ile Tyr Arg Thr Ser Asn Leu Ala Ser Gly Val Pro Ala
            180                 185                 190

Arg Phe Ser Gly Ser Gly Ser Gly Thr Ser Tyr Ser Leu Thr Ile Gly
        195                 200                 205

Thr Met Glu Ala Glu Asp Val Ala Thr Tyr Tyr Cys Gln Gln Gly Ser
    210                 215                 220

Ser Ile Pro Tyr Thr Phe Gly Gly Gly Thr Lys Leu Glu Leu Lys Asp
225                 230                 235                 240

Pro Ala Glu Pro Lys Ser Pro Asp Lys Thr His Thr Cys Pro Pro Cys
                245                 250                 255

Pro Ala Pro Glu Leu Leu Gly Gly Pro Ser Val Phe Leu Phe Pro Pro
            260                 265                 270

Lys Pro Lys Asp Thr Leu Met Ile Ser Arg Thr Pro Glu Val Thr Cys
        275                 280                 285

Val Val Val Asp Val Ser His Glu Asp Pro Glu Val Lys Phe Asn Trp
    290                 295                 300

Tyr Val Asp Gly Val Glu Val His Asn Ala Lys Thr Lys Pro Arg Glu
305                 310                 315                 320

Glu Gln Tyr Asn Ser Thr Tyr Arg Val Val Ser Val Leu Thr Val Leu
                325                 330                 335

His Gln Asp Trp Leu Asn Gly Lys Glu Tyr Lys Cys Lys Val Ser Asn
            340                 345                 350

Lys Ala Leu Pro Ala Pro Ile Glu Lys Thr Ile Ser Lys Ala Lys Gly
        355                 360                 365

Gln Pro Arg Glu Pro Gln Val Tyr Thr Leu Pro Pro Ser Arg Asp Glu
    370                 375                 380

Leu Thr Lys Asn Gln Val Ser Leu Thr Cys Leu Val Lys Gly Phe Tyr
385                 390                 395                 400

Pro Ser Asp Ile Ala Val Glu Trp Glu Ser Asn Gly Gln Pro Glu Asn
                405                 410                 415

Asn Tyr Lys Thr Thr Pro Pro Val Leu Asp Ser Asp Gly Ser Phe Phe
            420                 425                 430

Leu Tyr Ser Lys Leu Thr Val Asp Lys Ser Arg Trp Gln Gln Gly Asn
        435                 440                 445

Val Phe Ser Cys Ser Val Met His Glu Ala Leu His Asn His Tyr Thr
    450                 455                 460

Gln Lys Ser Leu Ser Leu Ser Pro Gly Lys Lys Asp Pro Lys Phe Trp

465                 470                 475                 480

Val Leu Val Val Val Gly Gly Val Leu Ala Cys Tyr Ser Leu Leu Val
                485                 490                 495

Thr Val Ala Phe Ile Ile Phe Trp Val Arg Ser Lys Arg Ser Arg Leu
            500                 505                 510

Leu His Ser Asp Tyr Met Asn Met Thr Pro Arg Arg Pro Gly Pro Thr
        515                 520                 525

Arg Lys His Tyr Gln Ala Tyr Ala Ala Ala Arg Asp Phe Ala Ala Tyr
    530                 535                 540

Arg Ser Leu Arg Val Lys Phe Ser Arg Ser Ala Asp Ala Pro Ala Tyr
545                 550                 555                 560

Gln Gln Gly Gln Asn Gln Leu Tyr Asn Glu Leu Asn Leu Gly Arg Arg
                565                 570                 575

Glu Glu Tyr Asp Val Leu Asp Lys Arg Arg Gly Arg Asp Pro Glu Met
            580                 585                 590

Gly Gly Lys Pro Arg Arg Lys Asn Pro Gln Glu Gly Leu Tyr Asn Glu
        595                 600                 605

Leu Gln Lys Asp Lys Met Ala Glu Ala Tyr Ser Glu Ile Gly Met Lys
    610                 615                 620

Gly Glu Arg Arg Arg Gly Lys Gly His Asp Gly Leu Tyr Gln Gly Leu
625                 630                 635                 640

Ser Thr Ala Thr Lys Asp Thr Tyr Asp Ala Leu His Met Gln Ala Leu
                645                 650                 655

Pro Pro Arg

<210>  7
<211>  717
<212>  DNA
<213>  Artificial Sequence

<220>
<223>  anti-CD25 scFv

<400>  7
caggtgaagc tgcagcagtc tggggctgtg ctggcaaggc ctggggcttc cgtgaagatg        60

tcctgcaagg cttctggcta caggtttacc aactactgga tgcactgggt aaaacagagg       120

cctggacagg gtctagaatg gattggtgtt atttatcctg gaaatagtga tactagctac 180

aaccagaagt tcaagggcaa ggccaaactg actgcagtca catccgccag cactgcctac 240

atggagctca gcagcctgac aaatgaggac tctgcggtct attactgtac aagagaggga 300

gaaggctctg actactgggg ccaaggcacc acggtcaccg tctcctcagg tggaggcggt 360

tcaggcggag gtggctctgg cggtggcgga tcgcaaattg ttctcaccca gtctccagca 420

accatggctg catctcccgg ggagaagatc actatcacct gcagtgccag ctcaagtata 480

agttccaatt acttgcattg gtatcagcag aagccaggat tctcccctaa actcttgatt 540

tataggactt ccaatctggc ttctggagtc ccagctcgct tcagtggcag tgggtctggg 600

acctcttact ctctcacaat tggcaccatg gaggctgaag atgttgccac ttactactgc 660

cagcagggta gtagtatacc gtacacgttc ggagggggga ccaagctgga gctgaag 717

<210> 8
<211> 708
<212> DNA
<213> Artificial Sequence

<220>
<223> linker domain human IgG1 Fc

<400> 8
gatcccgccg agcccaaatc tcctgacaaa actcacacat gcccaccgtg cccagcacct 60

gaactcctgg ggggaccgtc agtcttcctc ttccccccaa aacccaagga caccctcatg 120

atctcccgga cccctgaggt cacatgcgtg gtggtggacg tgagccacga agaccctgag 180

gtcaagttca actggtacgt ggacggcgtg gaggtgcata atgccaagac aaagccgcgg 240

gaggagcagt acaacagcac gtaccgggtg gtcagcgtcc tcaccgtcct gcaccaggac 300

tggctgaatg gcaaggagta caagtgcaag gtctccaaca aagccctccc agcccccatc 360

gagaaaacca tctccaaagc caaagggcag ccccgagaac cacaggtgta caccctgccc 420

ccatcccggg atgagctgac caagaaccag gtcagcctga cctgcctggt caaaggcttc 480

tatcccagcg acatcgccgt ggagtgggag agcaatgggc agccggagaa caactacaag 540

accacgcctc ccgtgctgga ctccgacggc tccttcttcc tctacagcaa gctcaccgtg 600

gacaagagca ggtggcagca ggggaacgtc ttctcatgct ccgtgatgca tgaggctctg 660

cacaaccact acacgcagaa gagcctctcc ctgtctccgg gtaaaaaa 708

<210> 9
<211> 702
<212> DNA
<213> Artificial Sequence

<220>
<223> linker domain human IgG1 delta Fc

<400>  9
cccgccgagc ccaaatctcc tgacaaaact cacacatgcc caccgtgccc agcacctcca      60

gtcgcgggac cgtcagtctt cctcttcccc ccaaaaccca aggacaccct catgatcgcc     120

cggacccctg aggtcacatg cgtggtggtg gacgtgagcc acgaagaccc tgaggtcaag     180

ttcaactggt acgtggacgg cgtggaggtg cataatgcca agacaaagcc gcgggaggag     240

cagtacaaca gcacgtaccg tgtggtcagc gtcctcaccg tcctgcacca ggactggctg     300

aatggcaagg agtacaagtg caaggtctcc aacaaagccc tcccagcccc catcgagaaa     360

accatctcca aagccaaagg gcagccccga gaaccacagg tgtacaccct gcccccatcc     420

cgggatgagc tgaccaagaa ccaggtcagc ctgacctgcc tggtcaaagg cttctatccc     480

agcgacatcg ccgtggagtg ggagagcaat gggcagccgg agaacaacta caagaccacg     540

cctcccgtgc tggactccga cggctccttc ttcctctaca gcaagctcac cgtggacaag     600

agcaggtggc agcaggggaa cgtcttctca tgctccgtga tgcatgaggc tctgcacaac     660

cactacacgc agaagagcct ctccctgtct ccgggtaaaa aa                        702

<210>  10
<211>  1899
<212>  DNA
<213>  Artificial Sequence

<220>
<223>  CAR # 1035, including leader sequence

<400>  10
atggattttc aggtgcagat tttcagcttc ctgctaatca gtgcctcagt cataatgtct      60

agacaggtga agctgcagca gtctgggact gtgctggcaa ggcctggggc ttccgtgaag     120

atgtcctgca aggcttctgg ctacaggttt accaactact ggatgcactg ggtaaaacag     180

aggcctggac agggtctaga atggattggt gttatttatc ctggaaatag tgatactagc     240

tacaaccaga agttcaaggg caaggccaaa ctgactgcag tcacatccgc cagcactgcc     300

tacatggagc tcagcagcct gacaaatgag gactctgcgg tctattactg tacaagagag     360

ggagaaggct ctgactactg gggccaaggg accacggtca ccgtctcctc aggtggaggc     420

ggttcaggcg gaggtggctc tggcggtggc ggatcgcaaa ttgttctcac ccagtctcca     480

gcaaccatgg ctgcatctcc cggggagaag atcactatca cctgcagtgc cagctcaagt     540

ataagttcca attacttgca ttggtatcag cagaagccag gattctcccc taaactcttg     600

atttatagga cttccaatct ggcttctgga gtcccagctc gcttcagtgg cagtgggtct     660

gggacctctt actctctcac aattggcacc atggaggctg aagatgttgc cacttactac     720

tgccagcagg gtagtagtat accgtacacg ttcggagggg ggaccaagct ggagctgaag     780

gatcccgccg agcccaaatc tcctgacaaa actcacacat gcccaccgtg cccagcacct     840

gaactcctgg ggggaccgtc agtcttcctc ttccccccaa aacccaagga caccctcatg 900

atctcccgga cccctgaggt cacatgcgtg gtggtggacg tgagccacga agaccctgag 960

gtcaagttca actggtacgt ggacggcgtg gaggtgcata atgccaagac aaagccgcgg 1020

gaggagcagt acaacagcac gtaccgggtg gtcagcgtcc tcaccgtcct gcaccaggac 1080

tggctgaatg gcaaggagta caagtgcaag gtctccaaca aagccctccc agcccccatc 1140

gagaaaacca tctccaaagc caaagggcag ccccgagaac cacaggtgta caccctgccc 1200

ccatcccggg atgagctgac caagaaccag gtcagcctga cctgcctggt caaaggcttc 1260

tatcccagcg acatcgccgt ggagtgggag agcaatgggc agccggagaa caactacaag 1320

accacgcctc ccgtgctgga ctccgacggc tccttcttcc tctacagcaa gctcaccgtg 1380

gacaagagca ggtggcagca ggggaacgtc ttctcatgct ccgtgatgca tgaggctctg 1440

cacaaccact acacgcagaa gagcctctcc ctgtctccgg gtaaaaaaga tcccaaactc 1500

tgctacctgc tggatggaat cctcttcatc tatggtgtca ttctcactgc cttgttcctg 1560

agagtgaagt tcagcaggag cgcagacgcc cccgcgtacc agcagggcca gaaccagctc 1620

tataacgagc tcaatctagg acgaagagag gagtacgatg ttttggacaa gagacgtggc 1680

cgggaccctg agatgggggg aaagccgaga aggaagaacc ctcaggaagg cctgtacaat 1740

gaactgcaga aagataagat ggcggaggcc tacagtgaga ttgggatgaa aggcgagcgc 1800

cggaggggca aggggcacga tggcctttac cagggtctca gtacagccac caaggacacc 1860

tacgacgccc ttcacatgca ggccctgccc cctcgctaa 1899

<210> 11
<211> 2043
<212> DNA
<213> Artificial Sequence

<220>
<223> CAR # 1036, including leader sequence

<400> 11
atggattttc aggtgcagat tttcagcttc ctgctaatca gtgcctcagt cataatgtct 60

agacaggtga agctgcagca gtctgggact gtgctggcaa ggcctggggc ttccgtgaag 120

atgtcctgca aggcttctgg ctacaggttt accaactact ggatgcactg ggtaaaacag 180

aggcctggac agggtctaga atggattggt gttatttatc ctggaaatag tgatactagc 240

tacaaccaga agttcaaggg caaggccaaa ctgactgcag tcacatccgc cagcactgcc 300

tacatggagc tcagcagcct gacaaatgag gactctgcgg tctattactg tacaagagag 360

ggagaaggct ctgactactg gggccaaggg accacggtca ccgtctcctc aggtggaggc 420

ggttcaggcg gaggtggctc tggcggtggc ggatcgcaaa ttgttctcac ccagtctcca 480

gcaaccatgg ctgcatctcc cggggagaag atcactatca cctgcagtgc cagctcaagt 540

ataagttcca attacttgca ttggtatcag cagaagccag gattctcccc taaactcttg 600

atttatagga cttccaatct ggcttctgga gtcccagctc gcttcagtgg cagtgggtct 660

gggacctctt actctctcac aattggcacc atggaggctg aagatgttgc cacttactac 720

tgccagcagg gtagtagtat accgtacacg ttcggagggg ggaccaagct ggagctgaag 780

gatcccgccg agcccaaatc tcctgacaaa actcacacat gcccaccgtg cccagcacct 840

gaactcctgg ggggaccgtc agtcttcctc ttccccccaa aacccaagga caccctcatg 900

atctcccgga cccctgaggt cacatgcgtg gtggtggacg tgagccacga agaccctgag 960

gtcaagttca actggtacgt ggacggcgtg gaggtgcata atgccaagac aaagccgcgg 1020

gaggagcagt acaacagcac gtaccgggtg gtcagcgtcc tcaccgtcct gcaccaggac 1080

tggctgaatg gcaaggagta caagtgcaag gtctccaaca aagccctccc agcccccatc 1140

gagaaaacca tctccaaagc caaagggcag ccccgagaac cacaggtgta caccctgccc 1200

ccatcccggg atgagctgac caagaaccag gtcagcctga cctgcctggt caaaggcttc 1260

tatcccagcg acatcgccgt ggagtgggag agcaatgggc agccggagaa caactacaag 1320

accacgcctc ccgtgctgga ctccgacggc tccttcttcc tctacagcaa gctcaccgtg 1380

gacaagagca ggtggcagca ggggaacgtc ttctcatgct ccgtgatgca tgaggctctg 1440

cacaaccact acacgcagaa gagcctctcc ctgtctccgg gtaaaaaaga tcccaaattt 1500

tgggtgctgg tggtggttgg tggagtcctg gcttgctata gcttgctagt aacagtggcc 1560

tttattattt tctgggtgag gagtaagagg agcaggctcc tgcacagtga ctacatgaac 1620

atgactcccc gccgccccgg gcccacccgc aagcattacc agccctatgc cccaccacgc 1680

gacttcgcag cctatcgctc cctgagagtg aagttcagca ggagcgcaga cgcccccgcg 1740

taccagcagg gccagaacca gctctataac gagctcaatc taggacgaag agaggagtac 1800

gatgttttgg acaagagacg tggccgggac cctgagatgg ggggaaagcc gagaaggaag 1860

aaccctcagg aaggcctgta caatgaactg cagaaagata agatggcgga ggcctacagt 1920

gagattggga tgaaaggcga gcgccggagg ggcaaggggc acgatggcct ttaccagggt 1980

ctcagtacag ccaccaagga cacctacgac gcccttcaca tgcaggccct gccccctcgc 2040

taa 2043

<210> 12
<211> 2043
<212> DNA
<213> Artificial Sequence

<220>
<223> CAR # 1037, including leader sequence

<400> 12
atggattttc aggtgcagat tttcagcttc ctgctaatca gtgcctcagt cataatgtct 60

agacaggtga agctgcagca gtctgggact gtgctggcaa ggcctggggc ttccgtgaag 120

atgtcctgca aggcttctgg ctacaggttt accaactact ggatgcactg ggtaaaacag 180

aggcctggac agggtctaga atggattggt gttatttatc ctggaaatag tgatactagc 240

tacaaccaga agttcaaggg caaggccaaa ctgactgcag tcacatccgc cagcactgcc 300

tacatggagc tcagcagcct gacaaatgag gactctgcgg tctattactg tacaagagag 360

ggagaaggct ctgactactg gggccaaggg accacggtca ccgtctcctc aggtggaggc 420

ggttcaggcg gaggtggctc tggcggtggc ggatcgcaaa ttgttctcac ccagtctcca 480

gcaaccatgg ctgcatctcc cggggagaag atcactatca cctgcagtgc cagctcaagt 540

ataagttcca attacttgca ttggtatcag cagaagccag gattctcccc taaactcttg 600

atttatagga cttccaatct ggcttctgga gtcccagctc gcttcagtgg cagtgggtct 660

gggacctctt actctctcac aattggcacc atggaggctg aagatgttgc cacttactac 720

tgccagcagg gtagtagtat accgtacacg ttcggagggg ggaccaagct ggagctgaag 780

gatcccgccg agcccaaatc tcctgacaaa actcacacat gcccaccgtg cccagcacct 840

gaactcctgg ggggaccgtc agtcttcctc ttccccccaa aacccaagga caccctcatg 900

atctcccgga cccctgaggt cacatgcgtg gtggtggacg tgagccacga agaccctgag 960

gtcaagttca actggtacgt ggacggcgtg gaggtgcata atgccaagac aaagccgcgg 1020

gaggagcagt acaacagcac gtaccgggtg gtcagcgtcc tcaccgtcct gcaccaggac 1080

tggctgaatg gcaaggagta caagtgcaag gtctccaaca aagccctccc agcccccatc 1140

gagaaaacca tctccaaagc caaagggcag ccccgagaac cacaggtgta caccctgccc 1200

ccatcccggg atgagctgac caagaaccag gtcagcctga cctgcctggt caaaggcttc 1260

tatcccagcg acatcgccgt ggagtgggag agcaatgggc agccggagaa caactacaag 1320

accacgcctc ccgtgctgga ctccgacggc tccttcttcc tctacagcaa gctcaccgtg 1380

gacaagagca ggtggcagca ggggaacgtc ttctcatgct ccgtgatgca tgaggctctg 1440

cacaaccact acacgcagaa gagcctctcc ctgtctccgg gtaaaaaaga tcccaaattt 1500

tgggtgctgg tggtggttgg tggagtcctg gcttgctata gcttgctagt aacagtggcc 1560

tttattattt tctgggtgag gagtaagagg agcaggctcc tgcacagtga ctacatgaac 1620

atgactcccc gccgccccgg gcccacccgc aagcattacc aggcctatgc cgccgcacgc 1680

gacttcgcag cctatcgctc cctgagagtg aagttcagca ggagcgcaga cgcccccgcg 1740

taccagcagg gccagaacca gctctataac gagctcaatc taggacgaag agaggagtac 1800

gatgttttgg acaagagacg tggccgggac cctgagatgg ggggaaagcc gagaaggaag 1860

aaccctcagg aaggcctgta caatgaactg cagaaagata agatggcgga ggcctacagt 1920

gagattggga tgaaaggcga gcgccggagg ggcaaggggc acgatggcct ttaccagggt 1980

ctcagtacag ccaccaagga cacctacgac gcccttcaca tgcaggccct gccccctcgc 2040

taa 2043

<210> 13
<211> 41
<212> PRT
<213> Artificial Sequence

<220>
<223> CD28 delta Lck intracellular domain

<400> 13

Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Ala Tyr Ala Ala
            20                  25                  30

Ala Arg Asp Phe Ala Ala Tyr Arg Ser
        35                  40

<210> 14
<211> 123
<212> DNA
<213> Artificial Sequence

<220>
<223> encoding SEQ ID NO. 13

<400> 14
aggagtaaga ggagcaggct cctgcacagt gactacatga acatgactcc ccgccgcccc 60

gggcccaccc gcaagcatta ccaggcctat gccgccgcac gcgacttcgc agcctatcgc 120

tcc 123

## Description

The present invention relates to proteins which comprise (i) a CD25-specific binding domain, (ii) a linker domain, connecting domain (i) and domain (iii), (iii) a transmembrane domain, and (iv) a signalling domain. The present invention furthermore relates to nucleic acids encoding the proteins, expression constructs for expressing the protein in a host cell and host cells. The present invention further relates to pharmaceutical compositions comprising said protein(s), nucleic acid(s), expression construct(s) or host cell(s). The proteins of the invention are CD25-specific chimeric antigen receptors that are suitable for generating CD25-specific immune cells, which can be used e.g. in the treatment of inflammation.

### BACKGROUND OF THE INVENTION

Auto-immune reaction has been found to be an underlying cause in many diseases; chronic inflammation is the main consequence of an auto-immune reaction and occurs when the auto-immune reaction is not limited and out of control. Numerous diseases are associated with chronic inflammation with a lasting and de-regulated activation of the cellular immune response. More than 80 auto-immune diseases exist; these are, for instance, multiple sclerosis, inflammatory bowel disease including Crohn's disease and ulcerative colitis, type-1 diabetes, psoriasis, rheumatoid arthritis, systemic lupus erytematosus, Hashimoto's thyreoiditis, Addison's disease, Graves' disease, Sjögren's syndrome, myastenia gravis, auto-immune vasculitis, pernicious anemia, and celiac disease.

Regulatory T cells (Treg cells) inhibit sustained inflammatory reactions, which is, however, not very effective in case of auto-immune diseases; the inflammatory reaction continues despite the presence of Treg cells. Steroids are regularly used for a therapeutic reduction of chronic inflammatory reactions. They, however, inhibit the inflammation only in an insufficient manner and have severe systemic effects.

Conventional treatment of chronic inflammatory diseases includes nonsteroidal anti-inflammatory drugs and immunosuppressing drugs like prednisone or cortisols that slow the immune cell attack and reduce inflammation; relapses are common after the drug is discontinued. In some auto-immune diseases such as Hashimoto's thyreoditis immunosuppressive drugs are usually not prescribed because the side effects outweight the benefits.

Thus, there is a need to inhibit deregulated inflammatory immune response at the site of inflammation in a targeted and lasting manner.

### SUMMARY OF THE INVENTION

According to the present invention this object is solved by a protein comprising
(i) a CD25-specific binding domain, preferably comprising an anti-CD25 single chain Fv (scFv) fragment;
(ii) a linker domain, connecting domain (i) and domain (iii), preferably comprising a human immunoglobulin Fc domain, more preferably comprising human IgG1 Fc;
(iii) a transmembrane domain, and
(iv) a signalling domain,
   wherein the signalling domain comprises
   a primary human signalling chain,
      preferably derived from human CD3 zeta chain or human FcεRI gamma chain,
   an intracellular co-stimulatory signalling chain,
      preferably derived from human CD28, 4-1BB, OX40 or CD27, or
   a fusion of said intracellular co-stimulatory signal chain(s) with the intracellular domain of a primary human signalling chain.

The protein of the present invention is a chimeric antigen receptor (CAR).

According to the present invention this object is furthermore solved by a nucleic acid encoding the CAR.

According to the present invention this object is furthermore solved by an expression construct for expressing the CAR.

According to the present invention this object is furthermore solved by a host cell expressing the CAR or comprising the nucleic acid or the expression construct.

According to the present invention this object is furthermore solved by a pharmaceutical composition, comprising
(i) at least one protein, nucleic acid, expression construct or host cell of the present invention, and
(ii) optionally, pharmaceutically acceptable excipient(s) and/or carrier, preferably harbouring (i).

According to the present invention this object is furthermore solved by using the CAR protein, nucleic acid, or expression construct for generating CD25-specific immune cells.

According to the present invention this object is furthermore solved by the CAR protein, nucleic acid, expression construct or host cell for use as a medicament.

According to the present invention this object is furthermore solved by the CAR protein, nucleic acid, expression construct or host cell for use in the treatment of inflammation.

According to the present invention this object is furthermore solved by the CAR protein, nucleic acid, expression construct or host cell for use in target-cell specific immunotherapy.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 21" should be interpreted to include not only the explicitly recited values of 1 to 21, but also include individual values and sub-ranges within the indicated range. Thus, included in this numerical range are individual values such as 1, 2, 3, 4, 5 .... 17, 18, 19, 20, 21 and sub-ranges such as from 2 to 10, 8 to 15, etc. This same principle applies to ranges reciting only one numerical value, such as "at least 90%". Furthermore, such an interpretation should apply regardless of the breadth of the range or the characteristics being described.

### CD25-specific CARs

As outlined above, the present invention provides CD25-specific chimeric antigen receptors (CARs).

The present invention provides a multi-domain or modular protein comprising
(i) a CD25-specific binding domain,
(ii) a linker domain, connecting domain (i) and domain (iii),
(iii) a transmembrane domain, and
(iv) a signalling domain,
   wherein the signalling domain comprises
   a primary human signalling chain,
      preferably derived from human CD3 zeta chain or human FcεRI gamma chain,
   an intracellular co-stimulatory signalling chain,
      preferably derived from human CD28, 4-1BB, OX40 or CD27, or
   a fusion of said intracellular co-stimulatory signal chain(s) with the intracellular domain of a primary human signalling chain.

The proteins of the invention are preferably cell surface receptor proteins and, thus, comprise an extracellular portion (domains (i) and (ii)), a transmembrane portion (domain (iii)) and a cytoplasmic portion (domain (iv)), and can thus be inserted into the outer cell membrane of the host cell. The functionality of the proteins of the invention within a host cell is detectable in an assay suitable for demonstrating the signaling potential of said protein upon binding of a particular ligand. Such assays are available to the skilled artisan.

Upon binding to the CD25, i.e. the target, such chimeric antigen receptors link to endogenous signalling pathways in a cell (an immune cell) and generate certain activating signals (depending on the signalling domain).

### (i) CD25-specific binding domain

CD25 is the α-chain of the interleukin 2 (IL-2) receptor, and when expressed with α- and β-chain, the receptor acquires high affinity for IL-2. CD25 is expressed on the surface of immune cells, preferably on activated immune cells.

The binding domain serves for the targeting of the protein according to the present invention or a respective cell expressing/carrying the protein according to the present invention on its surface to a target cell carrying CD25 on its surface.

Binding of the binding domain of the CAR to its cognate CD25 target on the surface of target cells furthermore results in transmitting a signal into the CAR-expressing immune cells via the intracellular signalling domain(s) of the CAR protein which activates the immune cell to execute a variety of effector functions including amplification, cytokine release, target cell elimination or repression and others.

A (target) binding domain of a CAR is usually derived from an antigen binding domain derived from an antibody against an antigen or receptor of the target, or a peptide binding an antigen or receptor of the target, or a peptide or protein ligand binding a receptor on the target.

In an embodiment, where domain (i) is derived from an antigen binding domain, the antigen binding domain is preferably derived from an antibody or an antibody fragment, such as a single chain Fv (scFv) fragment, a Fab fragment, a diabody, a variable domain of the antibody heavy chain or antibody light chain, a DARPin, an anticalin or any peptide or protein with specificity in binding to CD25.

In one embodiment, the extracellular CD25-specific binding domain (i) is derived from or comprises or consists of IL-2.

In a preferred embodiment, the CD25-specific binding domain (i) comprises or consists of an anti-CD25 single chain Fv (scFv) fragment.

In one embodiment, CD25-specific binding domain (i) comprises or consists of the amino acid sequence of SEQ ID NO. 1 [= amino acid sequence of the anti-CD25 scFv].

QVKLQQSGTVLARPGASVKMSCKASGYRFTNYWMHWVKQRPGQGLEWIGVIYPGNSDTSYNQ

KFKGKAKLTAVTSASTAYMELSSLTNEDSAVYYCTREGEGSDYWGQGTTVTVSSGGGGSGGG

GSGGGGSQIVLTQSPATMAASPGEKITITCSASSSISSNYLHWYQQKPGFSPKLLIYRTSNL

ASGVPARFSGSGSGTSYSLTIGTMEAEDVATYYCQQGSSIPYTFGGGTKLELK

### (ii) Linker domain

The linker domain or linker region (ii) connects the binding domain (i) and the transmembrane domain (iii).

The linker region serves as a spacer between the binding domain (i) and the transmembrane domain (iii).

In a preferred embodiment, the linker domain (ii) comprises a human immunoglobulin Fc domain, more preferably comprises or consists of human IgG1 Fc domain.

The IgG1 Fc domain may comprise the hinge-CH1-CH2-CH3 domain or parts thereof.

In one embodiment, the linker domain (ii) comprises or consists of the amino acid sequence of SEQ ID NO. 2 [= amino acid sequence of the linker domain IgG1-Fc]:

DPAEPKSPDKTHTCPPCPAPELLGGPSVFLFPPKPKDTLMISRTPEVTCVVVDVSHEDPEVK

FNWYVDGVEVHNAKTKPREEQYNSTYRVVSVLTVLHQDWLNGKEYKCKVSNKALPAPIEKTI

SKAKGQPREPQVYTLPPSRDELTKNQVSLTCLVKGFYPSDIAVEWESNGQPENNYKTTPPVL

DSDGSFFLYSKLTVDKSRWQQGNVFSCSVMHEALHNHYTQKSLSLSPGKK

The IgG1 Fc domain may comprise the hinge-CHI-CH2-CH3 domain or parts thereof. The domain may furthermore harbour a mutation to diminish binding to the Fc receptor. The mutation is described in Hombach *et al.* (2010), and comprises the amino acid residues PPVA-G(232-237) (...) IAR(253-255), see SEQ ID NO. 3 below, instead of the amino acid residues PELLGG(232-237) (...) ISR(253-255), see SEQ ID NO. 2 above.

In this embodiment, the linker domain (ii) comprises or consists of the amino acid sequence of SEQ ID NO. 3 [= amino acid sequence of the linker domain IgG1-ΔFc]:

PAEPKSPDKTHTCPPCPAPPVAGPSVFLFPPKPKDTLMIARTPEVTCVVVDVSHEDPEVKFN

WYVDGVEVHNAKTKPREEQYNSTYRVVSVLTVLHQDWLNGKEYKCKVSNKALPAPIEKTISK

AKGQPREPQVYTLPPSRDELTKNQVSLTCLVKGFYPSDIAVEWESNGQPENNYKTTPPVLDS

DGSFFLYSKLTVDKSRWQQGNVFSCSVMHEALHNHYTQKSLSLSPGKK

### (iii) Transmembrane domain

The transmembrane domain or transmembrane region anchors the protein of the present invention on the cell membrane.

The transmembrane domain is preferably derived from CD4, CD8, CD3, CD28 or 4-1BB, more preferably CD28. Any other transmembrane domain or region can likewise be used.

### (iv) Signalling domain

The signalling domain (iv) comprises one or more intracellular signalling domains.

The signalling domain (iv) is suitable for activating immune cells

The signalling domain serves the coupling of the target/antigen recognition to the intracellular signalling machinery.

Binding of the CD25-specific binding domain (i) of the CAR to its cognate target CD25 on the surface of target cells furthermore transmits a signal into the CAR-expressing immune cells via the intracellular signalling domain(s) of the CAR which activates the cell-intrinsic activity of such immune cells.

The signalling domain (iv) comprises or consists of (is)
- a primary human signalling chain,
- intracellular co-stimulatory signalling chain(s), or
- a fusion of said intracellular co-stimulatory signalling chain(s) with the intracellular domain of a primary human signal chain

Said primary human signalling chain is preferably derived from
human CD3 zeta chain, or
human FcεRI gamma chain
or parts thereof.

Said intracellular co-stimulatory signalling chain(s), which can be part of the fusion, are preferably derived from human CD28, 4-1BB, OX40 or CD27, more preferably CD28.

In a preferred embodiment, the transmembrane domain (iii) is derived from the CD3 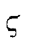 chain or the CD28 costimulatory molecule. Transmembrane domains from other molecules like CD4 or CD8 can likewise be used.

In a preferred embodiment, the signalling domain (iv) is derived or selected from
(1) the human CD3 zeta chain,
(2) the intracellular domain of human CD28 linked to the intracellular domain of human CD3 zeta chain, and
(3) the intracellular domain of human CD28 linked to the intracellular domain of human CD3 zeta chain, wherein CD28 comprises at least one mutation.

In a preferred embodiment, said mutation in CD28 is a deletion of the binding site for Lck (lymphocyte-specific protein kinase), such as described in SED ID NO. 13, which shows the CD28ΔLck intracellular domain:
RSKRSRLLHSDYMNMTPRRPGPTRKHYQAYAAARDFAAYRS

Deletion of the Lck binding site within the CD28 costimulatory domain of the CAR results in the induction of IL-2 release upon CAR signaling is substantially reduced. Other T cell effector functions initiated by the CAR are not affected like T cell amplification, IFN-g release or cytolytic activities.

### (v) Further domains or components

In one embodiment, the CAR of the present invention further comprises an N-terminal secretion signal (leader) peptide.

Said "secretion signal peptide" refers to a peptide sequence that directs the transport of the CAR of the invention to the cell membrane and cell surface. It, thus, allows correct localization of the CAR, in particular the extracellular portion (domains (i) and (ii)) on the cell surface; the transmembrane portion (domain (iii)) inserted into the plasma membrane and the cytoplasmic portion (domain (iv)) in the host cell.

In an embodiment, the secretion signal peptide comprises or is immunoglobulin heavy chain signal peptide, such as the IgG kappa light chain leader sequence.

In a preferred embodiment, the CAR comprises or consists of an amino acid sequence selected from SEQ ID NOs. 4 to 6 *[*= *full-length sequences of CAR #1035 to 1037],*
or an amino acid sequence that has at least 85%, preferably at least 90% or at least 95 % sequence identity to an amino acid sequence of SEQ ID NOs. 4 to 6.

The amino acid sequence of SEQ ID NO. 4 refers to the amino acid sequence of a CAR with the domains:
(i)[*anti-CD25 scFv (Rft5 scFv)*] - (ii)[IgG1 Fc] - (iii and iv)[transmembrane and intracellular domain of the human CD3 zeta chain]
CAR # 1035 SEQ ID NO. 4

QVKLQQSGTVLARPGASVKMSCKASGYRFTNYWMHWVKQRPGQGLEWIGVIYPGNSDTSYNQ
KFKGKAKLTAVTSASTAYMELSSLTNEDSAVYYCTREGEGSDYWGQGTTVTVSSGGGGSGGG
GSGGGGSQIVLTQSPATMAASPGEKITITCSASSSISSNYLHWYQQKPGFSPKLLIYRTSNL
ASGVPARFSGSGSGTSYSLTIGTMEAEDVATYYCQQGSSIPYTFGGGTKLELKDPAEPKSPD
KTHTCPPCPAPELLGGPSVFLFPPKPKDTLMISRTPEVTCVVVDVSHEDPEVKFNWYVDGVE

VHNAKTKPREEQYNSTYRVVSVLTVLHQDWLNGKEYKCKVSNKALPAPIEKTISKAKGQPRE

PQVYTLPPSRDELTKNQVSLTCLVKGFYPSDIAVEWESNGQPENNYKTTPPVLDSDGSFFLY

SKLTVDKSRWQQGNVFSCSVMHEALHNHYTQKSLSLSPGKKDPKLCYLLDGILFIYGVILTA

LFLRVKFSRSADAPAYQQGQNQLYNELNLGRREEYDVLDKRRGRDPEMGGKPRRKNPQEGLY

NELQKDKMAEAYSEIGMKGERRRGKGHDGLYQGLSTATKDTYDALHMQALPPR

The amino acid sequence of SEQ ID NO. 5 refers to the amino acid sequence of a CAR with the domains:
(i)[*anti-CD25 scFv (Rft5 scFv)*] - (ii)[IgG1 Fc] - (iii and iv)[fusion of the *transmembrane and intracellular domain of human CD28* with the intracellular domain of human CD3 zeta chain]
CAR # 1036 SEQ ID NO. 5

QVKLQQSGTVLARPGASVKMSCKASGYRFTNYWMHWVKQRPGQGLEWIGVIYPGNSDTSYNQ
KFKGKAKLTAVTSASTAYMELSSLTNEDSAVYYCTREGEGSDYWGQGTTVTVSSGGGGSGGG
GSGGGGSQIVLTQSPATMAASPGEKITITCSASSSISSNYLHWYQQKPGFSPKLLIYRTSNL
ASGVPARFSGSGSGTSYSLTIGTMEAEDVATYYCQQGSSIPYTFGGGTKLELKDPAEPKSPD
KTHTCPPCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSDGSFFLY
SKLTVDKSRWQQGNVFSCSVMHEALHNHYTQKSLSLSPGKKDPKFWVLVVVGGVLACYSLLV
TVAFIIFWVRSKRSRLLHSDYMNMTPRRPGPTRKHYQPYAPPRDFAAYRSLRVKFSRSADAP
AYQQGQNQLYNELNLGRREEYDVLDKRRGRDPEMGGKPRRKNPQEGLYNELQKDKMAEAYSE
IGMKGERRRGKGHDGLYQGLSTATKDTYDALHMQALPPR

The amino acid sequence of SEQ ID NO. 6 refers to the amino acid sequence of a CAR with the domains:
(i)[*anti-CD25 scFv (Rft5 scFv)*] - (ii)[IgG1 Fc] - (iii and iv)[fusion of the transmembrane and intracellular domain of human CD28, **comprising a deletion of the Lck binding site,** with the intracellular domain of human CD3 zeta chain]
CAR # 1037 SEQ ID NO. 6

QVKLQQSGTVLARPGASVKMSCKASGYRFTNYWMHWVKQRPGQGLEWIGVIYPGNSDTSYNQ
KFKGKAKLTAVTSASTAYMELSSLTNEDSAVYYCTREGEGSDYWGQGTTVTVSSGGGGSGGG
GSGGGGSQIVLTQSPATMAASPGEKITITCSASSSISSNYLHWYQQKPGFSPKLLIYRTSNL

ASGVPARFSGSGSGTSYSLTIGTMEAEDVATYYCQQGSSIPYTFGGGTKLELKDPAEPKSPD
KTHTCPPCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSDGSFFLY
SKLTVDKSRWQQGNVFSCSVMHEALHNHYTQKSLSLSPGKKDPKFWVLVVVGGVLACYSLLV
TVAFIIFWVRSKRSRLLHSDYMNMTPRRPGPTRKHYQAYAAARDFAAYRSLRVKFSRSADAP
AYQQGQNQLYNELNLGRREEYDVLDKRRGRDPEMGGKPRRKNPQEGLYNELQKDKMAEAYSE
IGMKGERRRGKGHDGLYQGLSTATKDTYDALHMQALPPR

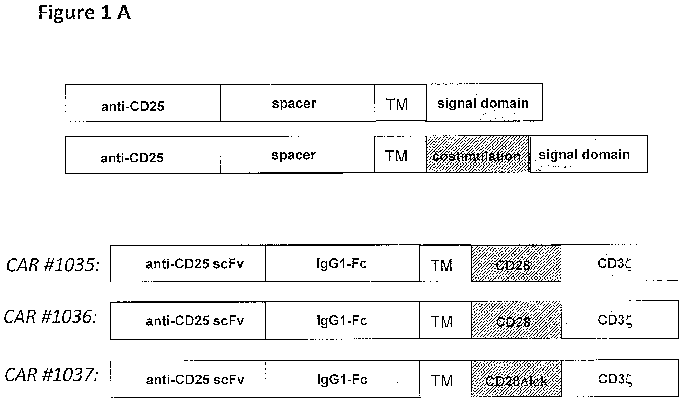

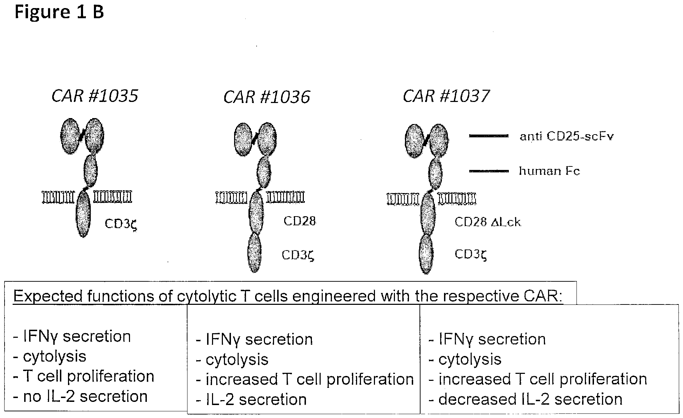

The CARs differ in their signalling domain (iv) and, thus, in their induced T cell effector functions, see e.g. Figure 1B.

CAR # 1035, comprising the human CD3 zeta chain, will preferably induce effector functions of engineered T cells including IFN-γ secretion and cytolysis of CD25+ target cells.

Preferably, a cytolytic T cell engineered with the CAR # 1035, comprising the human CD3 zeta chain, will recognize CD25+ target cells and as a consequence will release IFN-γ, lyse the CD25+ target cells and will amplify. CD25- cells are not recognized by CAR #1035 T cells and will not specifically induce T cell activation.

CAR # 1036, comprising a fusion of the transmembrane and intracellular domain of human CD28 with the intracellular domain of human CD3 zeta chain, will preferably induce effector functions of engineered T cells including IFN-γ secretion, release of IL-2 and cytolysis of CD25+ target cells.

Preferably, a cytolytic T cell engineered with the CAR # 1036, comprising a fusion of the transmembrane and intracellular domain of human CD28 with the intracellular domain of human CD3 zeta chain, will recognize CD25+ target cells and as a consequence will release IFN-γ and IL-2, lyse the CD25+ target cells and will amplify. CD25- cells are not recognized by CAR #1036 T cells and will not specifically induce T cell activation.

CAR # 1037, comprising a fusion of the transmembrane and intracellular domain of human CD28 (comprising a deletion of the Lck binding site) with the intracellular domain of human CD3 zeta chain, will preferably induce effector functions of engineered T cells including IFN-γ secretion, release of very low amounts of IL-2, and cytolysis of CD25+ target cells. Preferably, a cytolytic T cell engineered with the CAR # 1037, comprising a fusion of the transmembrane and intracellular domain of human CD28 (comprising a deletion of the Lck binding site) with the intracellular domain of human CD3 zeta chain, will recognize CD25+ target cells and as a consequence will release IFN-γ and very low amounts of IL-2, lyse the CD25+ target cells and will amplify. CD25- cells are not recognized by CAR #1037 T cells and will not specifically induce T cell activation.

### Nucleic acids, expression constructs and host cells

As described above, the present invention provides *nucleic acids*/nucleic acid molecules/isolated nucleic acid molecules encoding the proteins of the invention.

The nucleic acids according to this invention comprise DNA (such as dsDNA, ssDNA, cDNA), RNA (such as dsRNA, ssRNA, mRNA), combinations thereof or derivatives (such as PNA) thereof.

Preferably, a nucleic acid of the invention comprises
- the nucleic acid encoding for the amino acid sequence of SEQ ID NO. 1 or
- the nucleic acid sequence of SEQ ID NO. 7 [= nucleotide sequence of the anti-CD25 scFv],
or their complementary sequences
or sequences that have at least 85%, preferably at least 90% or at least 95 % sequence identity to the above sequences.

Preferably, a nucleic acid of the invention further comprises
- the nucleic acid encoding for the amino acid sequence of SEQ ID NO. 2 or 3 or
- the nucleic acid sequence of SEQ ID NO. 8 [= nucleotide sequence of the IgG1 Fc] or SEQ ID NO. 9 [= nucleotide sequence of the IgG1 ΔFc],
or their complementary sequences
or sequences that have at least 85%, preferably at least 90% or at least 95 % sequence identity to the above sequences.

Preferably, a nucleic acid of the invention comprises or consists of
- the nucleic acid encoding for an amino acid sequence selected from SEQ ID NOs. 4 to 6 or
- a nucleic acid sequence selected from SEQ ID NOs. 10 to 12 [= nucleotide sequence encoding the CAR #1035 to 1037, each including the leader peptide]
or their complementary sequences
or sequences that have at least 85%, preferably at least 90% or at least 95 % sequence identity.

Preferably, the nucleic acid sequences of the present invention are human sequences or codon-optimized for the expression in mammalian cells, preferably for the expression in human cells. Codon-optimization refers to the exchange in a sequence of interest of codons that are generally rare in highly expressed genes of a given species by codons that are generally frequent in highly expressed genes of such species, such codons encoding the same amino acids as the codons that are being exchanged.

Within the scope of this invention are also the nucleotide sequences obtained due to the degeneration of the genetic code of the above nucleotide sequences.

The nucleotide sequence of SEQ ID NO. 10 refers to the nucleotide sequence of a CAR with the domains:
[leader peptide] - (i)[*cmti-CD25 scFv (Rft5 scFv)*] - (ii)[IgG1 Fc] - (iii and
iv)[transmembrane and intracellular domain of the human CD3 zeta chain]
CAR # 1035 SEQ ID NO. 10

atggattttcaggtgcagattttcagcttcctgctaatcagtgcctcagtcataatgtctag
acaggtgaagctgcagcagtctggactgtgctggcaaggcctggggcttccgtgaagatgt
cctgcaaggcttctggctacaggtttaccaactactggatgcactgggtaaaacagaggcct
ggacagggtctagaatggattggtgttatttatcctggaaatagtgatactagctacaacca
gaagttcaagggcaaggccaaactgactgcagtcacatccgccagcactgcctacatggagc
tcagcagcctgacaaatgaggactctgcggtctattactgtacaagagagggagaaggctct
gactactggggccaaggaccacggtcaccgtctcctcaggtggaggcggttcaggcggagg
tggctctggcggtggcggatcgcaaattgttctcacccagtctccagcaaccatggctgcat
ctcccggggagaagatcactatcacctgcagtgccagctcaagtataagttccaattacttg
cattggtatcagcagaagccaggattctcccctaaactcttgatttataggacttccaatct
ggcttctggagtcccagctcgcttcagtggcagtgggtctgggacctcttactctctcacaa

ttggcaccatggaggctgaagatgttgccacttactactgccagcagggtagtagtataccg
tacacgttcggagggggaccaagctggagctgaaggatcccgccgagcccaaatctcctga
caaaactcacacatgcccaccgtgcccagcacctgaactcctggggggaccgtcagtcttcc
tcttccccccaaaacccaaggacaccctcatgatctcccggacccctgaggtcacatgcgtg
gtggtggacgtgagccacgaagaccctgaggtcaagttcaactggtacgtggacggcgtgga
ggtgcataatgccaagacaaagccgcgggaggagcagtacaacagcacgtaccgggtggtca
gcgtcctcaccgtcctgcaccaggactggctgaatggcaaggagtacaagtgcaaggtctcc
aacaaagccctcccagcccccatcgagaaaaccatctccaaagccaaagggcagccccgaga
accacaggtgtacaccctgcccccatcccgggatgagctgaccaagaaccaggtcagcctga
cctgcctggtcaaaggcttctatcccagcgacatcgccgtggagtgggagagcaatgggcag
ccggagaacaactacaagaccacgcctcccgtgctggactccgacggctccttcttcctcta
cagcaagctcaccgtggacaagagcaggtggcagcaggggaacgtcttctcatgctccgtga
tgcatgaggctctgcacaaccactacacgcagaagagcctctccctgtctccgggtaaaaaa
gatcccaaactctgctacctgctggatggaatcctcttcatctatggtgtcattctcactgc
cttgttcctgagagtgaagttcagcaggagcgcagacgcccccgcgtaccagcagggccaga
accagctctataacgagctcaatctaggacgaagagaggagtacgatgttttggacaagaga
cgtggccgggaccctgagatggggggaaagccgagaaggaagaaccctcaggaaggcctgta
caatgaactgcagaaagataagatggcggaggcctacagtgagattgggatgaaaggcgagc
gccggaggggcaaggggcacgatggcctttaccagggtctcagtacagccaccaaggacacc
tacgacgcccttcacatgcaggccctgccccctcgctaa

The nucleotide sequence of SEQ ID NO. 11 refers to the nucleotide sequence of a CAR with the domains:
[leader peptide] - (i)[a*nti-CD25 scFv (Rft5 scFv)*] - (ii)[IgG1 Fc] - (iii and iv)[fusion of the *transmembrane and intracellular domain of human CD28* with the intracellular domain of human CD3 zeta chain]
CAR # 1036 SEQ ID NO. 11

atggattttcaggtgcagattttcagcttcctgctaatcagtgcctcagtcataatgtctag
acaggtgaagctgcagcagtctggactgtgctggcaaggcctggggcttccgtgaagatgt
cctgcaaggcttctggctacaggtttaccaactactggatgcactgggtaaaacagaggcct
ggacagggtctagaatggattggtgttatttatcctggaaatagtgatactagctacaacca
gaagttcaagggcaaggccaaactgactgcagtcacatccgccagcactgcctacatggagc
tcagcagcctgacaaatgaggactctgcggtctattactgtacaagagagggagaaggctct
gactactggggccaaggaccacggtcaccgtctcctcaggtggaggcggttcaggcggagg
tggctctggcggtggcggatcgcaaattgttctcacccagtctccagcaaccatggctgcat
ctcccggggagaagatcactatcacctgcagtgccagctcaagtataagttccaattacttg
cattggtatcagcagaagccaggattctcccctaaactcttgatttataggacttccaatct
ggcttctggagtcccagctcgcttcagtggcagtgggtctgggacctcttactctctcacaa
ttggcaccatggaggctgaagatgttgccacttactactgccagcagggtagtagtataccg
tacacgttcggaggggggaccaagctggagctgaaggatcccgccgagcccaaatctcctga
caaaactcacacatgcccaccgtgcccagcacctgaactcctggggggaccgtcagtcttcc
tcttccccccaaaacccaaggacaccctcatgatctcccggacccctgaggtcacatgcgtg
gtggtggacgtgagccacgaagaccctgaggtcaagttcaactggtacgtggacggcgtgga
ggtgcataatgccaagacaaagccgcgggaggagcagtacaacagcacgtaccgggtggtca
gcgtcctcaccgtcctgcaccaggactggctgaatggcaaggagtacaagtgcaaggtctcc

aacaaagccctcccagcccccatcgagaaaaccatctccaaagccaaagggcagccccgaga
accacaggtgtacaccctgcccccatcccgggatgagctgaccaagaaccaggtcagcctga
cctgcctggtcaaaggcttctatcccagcgacatcgccgtggagtgggagagcaatgggcag
ccggagaacaactacaagaccacgcctcccgtgctggactccgacggctccttcttcctcta
cagcaagctcaccgtggacaagagcaggtggcagcaggggaacgtcttctcatgctccgtga
tgcatgaggctctgcacaaccactacacgcagaagagcctctccctgtctccgggtaaaaaa
gatcccaaatttgggtgctggtggtggttggtggagtcctggcttgctatagcttgctagt
aacagtggcctttattatttctgggtgaggagtaagaggagcaggctcctgcacagtgact
acatgaacatgactccccgccgccccgggcccacccgcaagcattaccagccctatgccccc
ccacgcgacttcgcagcctatcgctccctgagagtgaagttcagcaggagcgcagacgcccc
cgcgtaccagcagggccagaaccagctctataacgagctcaatctaggacgaagagaggagt
acgatgttttggacaagagacgtggccgggaccctgagatggggggaaagccgagaaggaag
aaccctcaggaaggcctgtacaatgaactgcagaaagataagatggcggaggcctacagtga
gattgggatgaaaggcgagcgccggaggggcaaggggcacgatggcctttaccagggtctca
gtacagccaccaaggacacctacgacgcccttcacatgcaggccctgccccctcgctaa

The nucleotide sequence of SEQ ID NO. 12 refers to the nucleotide sequence of a CAR with the domains:
[leader peptide] - (i)[*anti-CD25 scFv (Rft5 scFv)*] - (ii)[IgG1 Fc] - (iii and iv)[fusion of the *transmembrane and intracellular domain of human CD28,* **comprising a deletion of the Lck binding site,** with the intracellular domain of human CD3 zeta chain]
CAR # 1037 SEQ ID NO. 12

atggattttcaggtgcagattttcagcttcctgctaatcagtgcctcagtcataatgtctag
acaggtgaagctgcagcagtctggactgtgctggcaaggcctggggcttccgtgaagatgt
cctgcaaggcttctggctacaggtttaccaactactggatgcactgggtaaaacagaggcct
ggacagggtctagaatggattggtgttatttatcctggaaatagtgatactagctacaacca
gaagttcaagggcaaggccaaactgactgcagtcacatccgccagcactgcctacatggagc
tcagcagcctgacaaatgaggactctgcggtctattactgtacaagagagggagaaggctct
gactactggggccaagggaccacggtcaccgtctcctcaggtggaggcggttcaggcggagg
tggctctggcggtggcggatcgcaaattgttctcacccagtctccagcaaccatggctgcat
ctcccggggagaagatcactatcacctgcagtgccagctcaagtataagttccaattacttg
cattggtatcagcagaagccaggattctcccctaaactcttgatttataggacttccaatct
ggcttctggagtcccagctcgcttcagtggcagtgggtctgggacctcttactctctcacaa
ttggcaccatggaggctgaagatgttgccacttactactgccagcagggtagtagtataccg
tacacgttcggaggggggaccaagctggagctgaaggatcccgccgagcccaaatctcctga
caaaactcacacatgcccaccgtgcccagcacctgaactcctggggggaccgtcagtcttcc
tcttccccccaaaacccaaggacaccctcatgatctcccggacccctgaggtcacatgcgtg
gtggtggacgtgagccacgaagaccctgaggtcaagttcaactggtacgtggacggcgtgga
ggtgcataatgccaagacaaagccgcgggaggagcagtacaacagcacgtaccgggtggtca
gcgtcctcaccgtcctgcaccaggactggctgaatggcaaggagtacaagtgcaaggtctcc
aacaaagccctcccagcccccatcgagaaaaccatctccaaagccaaagggcagccccgaga
accacaggtgtacaccctgcccccatcccgggatgagctgaccaagaaccaggtcagcctga
cctgcctggtcaaaggcttctatcccagcgacatcgccgtggagtgggagagcaatgggcag
ccggagaacaactacaagaccacgcctcccgtgctggactccgacggctccttcttcctcta
cagcaagctcaccgtggacaagagcaggtggcagcaggggaacgtcttctcatgctccgtga
tgcatgaggctctgcacaaccactacacgcagaagagcctctccctgtctccgggtaaaaaa
gatcccaaatttgggtgctggtggtggttggtggagtcctggcttgctatagcttgctagt

aacagtggcctttattattttctgggtgaggagtaagaggagcaggctcctgcacagtgact
acatgaacatgactccccgccgccccgggcccacccgcaagcattaccaggcctatgccgcc
gcacgcgacttcgcagcctatcgctccctgagagtgaagttcagcaggagcgcagacgcccc
cgcgtaccagcagggccagaaccagctctataacgagctcaatctaggacgaagagaggagt
acgatgttttggacaagagacgtggccgggaccctgagatggggggaaagccgagaaggaag
aaccctcaggaaggcctgtacaatgaactgcagaaagataagatggcggaggcctacagtga
gattgggatgaaaggcgagcgccggaggggcaaggggcacgatggcctttaccagggtctca
gtacagccaccaaggacacctacgacgcccttcacatgcaggccctgccccctcgctaa

As described above, the present invention provides *expression constructs* for expressing the protein of the invention in a cell.

Preferably, the expression constructs further comprise promoter and terminator sequences.

An "expression or gene construct" (wherein both terms are used interchangeably throughout this specification) refers to a nucleic acid construct, usually an expression vector or plasmid that is used to introduce a specific gene or coding sequence into a target cell. Once the expression or gene construct is inside the cell, the encoded protein is produced by the cellular transcription and translation machinery. The expression or gene construct is designed to contain respective regulatory sequences that act as enhancer and promoter regions and lead to efficient transcription of the gene carried on the construct, including promoter and terminator sequences.

The skilled artisan can select further suitable components of expression or gene constructs.

An expression construct of the present invention is preferably transferred to T cells or other immune cells by γ-retroviral or lentiviral vectors. Alternatively, RNA transfer or DNA transfer by means of electroporation or other transfer methods known to the artisan are also applicable.

As described above, the present invention provides *host cells* which express a protein of the invention or which comprise a nucleic acid or an expression construct of the invention.

Preferably, the host cell is a cell of the immune system, more preferably T cells or regulatory T (Treg) cells; other cells like cells of the innate immune system like NK cells, macrophages, granulocytes can likewise be used as host cells.

### Uses and medical uses of the proteins, nucleic acids, expression constructs and host cells and pharmaceutical compositions

As described above, the invention provides the use of the protein (the CAR), nucleic acid, or expression construct for generating CD25-specific immune cells.

Preferably, the invention provides the use of the protein, nucleic acid, or expression construct for generating CD25-specific T cells or CD25-specific regulatory T (Treg) cells.

As described above, the present invention provides pharmaceutical compositions.

A pharmaceutical composition of the present invention comprises
(i) at least one protein of the present invention, at least one nucleic acid of the present invention, at least one expression construct of the present invention or at least one host cell of the present invention, and
(ii) optionally, pharmaceutically acceptable excipient(s) and/or carrier, preferably harbouring (i).

As described above, the present invention provides the protein (the CAR), the nucleic acid, the expression construct or the host cell for use as a medicament.

The invention provides the protein (the CAR), the nucleic acid, the expression construct or the host cell for use as a therapeutic product and/or pharmaceutical product.

As described above, the invention provides the protein (the CAR), the nucleic acid, the expression construct or the host cell for use in the treatment of inflammation.

An "inflammation" within the present invention refers to an immune cell response to stimulation by invading pathogens or endogenous signals such as damaged cells. Inflammation involves the recruitment and activation of a plethora of immune cells that results in tissue repair and return to homeostasis. As a result of local molecular, immunological and physiological processes, each tissue exhibits distinct mechanisms of inflammation, all involving immune cells or specific immune cell products like antibodies or cytokines. While physiological inflammation is self-limiting, under various pathological situations, inflammation results in uncontrolled immune cell activation, amplification and tissue damage. This is the case, for instance, during aging and senescence, during dysregulated neurological-immunological interactions, disturbance of cellular metabolism, interaction with the microbiome and others.

Preferably, "inflammation" comprises chronic inflammation, in particular in autoimmune diseases,
such as multiple sclerosis, inflammatory bowel disease including Crohn's disease and ulcerative colitis, type-1 diabetes, psoriasis, rheumatoid arthritis, systemic lupus erytematosus, Hashimoto's thyreoiditis, Addison's disease, Graves' disease, Sjögren's syndrome, myastenia gravis, auto-immune vasculitis, pernicious anemia, celiac disease, or others

Said treatment of inflammation preferably comprises reducing or supressing the inflammatory reaction in tissues.

As described above, the invention provides the protein (the CAR), the nucleic acid, the expression construct or the host cell for use in target-cell specific immunotherapy.

### Methods for treatment of inflammation

The present invention further provides a method for the treatment of inflammation, in particular in auto-immune diseases.

Said method according to the present invention comprises
administering to a subject in a therapeutically effective amount
(a) a protein, a nucleic acid, an expression construct or a host cell, as obtained and defined herein, and
(b) optionally, respective excipient(s).

Said host cell is preferably a host for the protein, nucleid acid or expression construct according to the present invention.

Said method preferably comprises reducing or supressing the inflammatory reaction in tissues.

The present invention further provides a method for targeting active immune cells.

Said method according to the present invention comprises
administering to a subject in a therapeutically effective amount
(a) a protein, a nucleic acid, an expression construct or a CD25-specific immune cell as obtained and defined herein, and
(b) optionally, respective excipient(s).

### Preferred embodiments

The present invention provides chimeric antigen receptors (CARs) which target CD25. T cells or Treg cells are provided with the anti-CD25 CARs. Such CAR-modified T cells contact active immune cells which carry CD25 on their surface and suppress inflammation at the inflammatory lesion.

CARs are recombinant transmembrane receptors which are assembled from different modules or domains and which are expressed in the surface of immune cells after gene transfer. Due to an antibody domain in their extracellular portion, CARs bind to defined target structures and convey through their intracellular portion an activation of the immune cell. Various CARs are described which are specific for tumor antigens and which target cytotoxic T cells against tumors. After binding the antigen, the cytolytic T cell is activated and lyses the cognate target cell.

This basic principle was adapted to a new concept in this invention. CARs for use in immune cells were designed and generated that target CD25 (the IL-2 receptor) on the surface of active immune cells. This is in contrast to current CARs that are designed to target tumor or other diseased cells.

In this application, we show anti-CD25 CARs which were transferred into cytotoxic T cells and suppressor Treg cells and expressed in these cells. The CAR-modified immune cells were used for suppressing an inflammatory reaction.

According to the invention, the anti-CD25 CAR-modified T cells eliminate the activated immune cells and, thus, stop the pro-inflammatory immune reaction. Since the CAR is not directed against a defined tissue, but activated immune cells, it can be used for the reduction of inflammation in any tissue.

The invention discloses, for the first time, the strategy of eliminating and suppressing inflammation cells via CAR-modified T cells which are specific for CD25. Applications are any inflammation of acute and chronic progression, in particular in the context of auto-immune diseases.

The following examples and drawings illustrate the present invention without, however, limiting the same thereto.

### BRIEF DESCRIPTION OF THE DRAWINGS

**Figure 1****.** *Design of the anti-CD25 CARs and their domain structure.*
   A shown is the modular structure of an anti-CD25 CAR according to the present invention and specific embodiments of anti-CD25 CARs.
   B, shown are three preferred embodiments of anti-CD25 CARs and their effect on T cell effector functions.

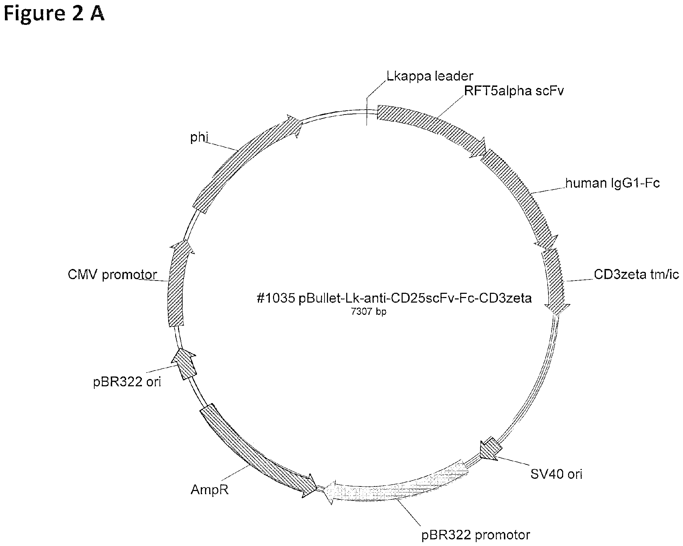

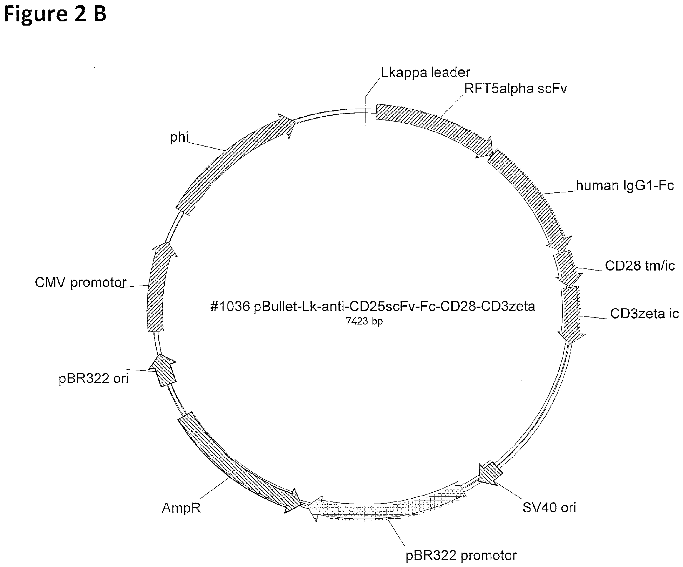

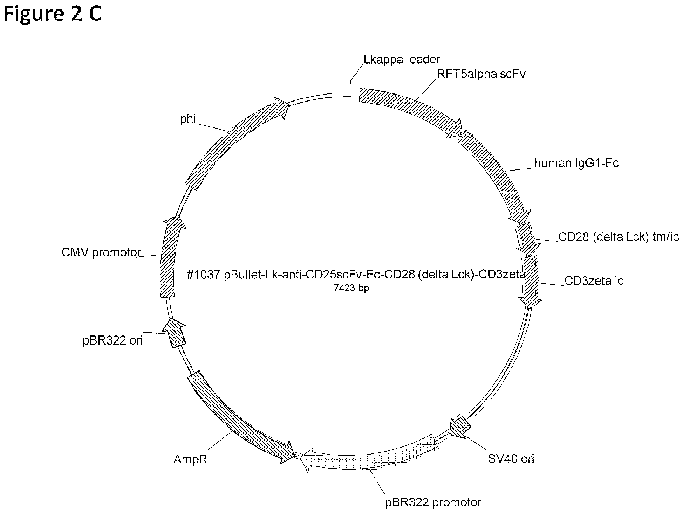

**Figure 2****.** *Expression constructs of three anti-CD25 CARs.*
   A, Plasmid map of anti-CD25 CAR #1035
   B, Plasmid map of anti-CD25 CAR #1036
   C, Plasmid map of anti-CD25 CAR #1037
**Figure 3****.** *Expression of the anti-CD25 CARs on human T cells.*
   Human T cells were retrovirally transduced with the expression constructs for anti-CD25 CAR #1035, anti-CD25 CAR #1036 and anti-CD25 CAR #1037, respectively. T cells without any CAR served as a control (d). After 48 hours, the CARs were detected by flow cytometry using a PE-conjugated anti-IgGl(Fc) antibody that recognizes the common extracellular IgG1(Fc) linker of the CARs. T cells were detected by staining with an anti-CD3 antibody.

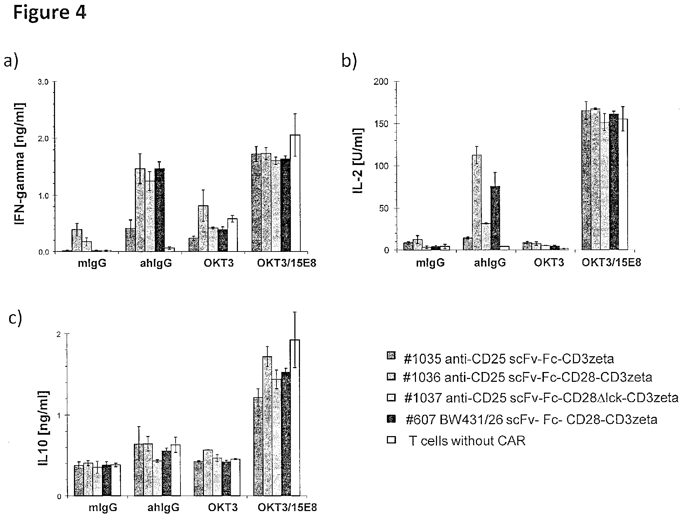

**Figure 4****.** *Specific activation of T cells.*
   Secretion of cytokines upon CAR stimulation of CAR T cells.
   Human T cells from the peripheral blood were retrovirally transduced with the expression constructs for the anti-CD25 CAR #1035, anti-CD25 CAR #1036 and anti-CD25 CAR #1037, respectively. Non-transduced T cells and T cells engineered with the anti-CEA CAR BW431/26-Fc-CD28-CD3 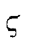 #607 served as controls. CAR T cells (10⁴ cells per well) were specifically stimulated for 48 hours by incubation on immobilized anti-human IgG1 antibody that recognizes the common extracellular IgG1 spacer. As controls, T cells were incubated with immobilized mouse IgG of irrelevant specificity (mIgG), immobilized agonistic anti-CD3 antibody OKT3, immobilized agonistic anti-CD28 antibody 15E8, or both immobilized OKT3 and 15E8, respectively. IFN-γ (a), IL-2 (b) and IL-10 in the culture supernatant were determined by ELISA. Data represent the mean ± standard error of the mean.

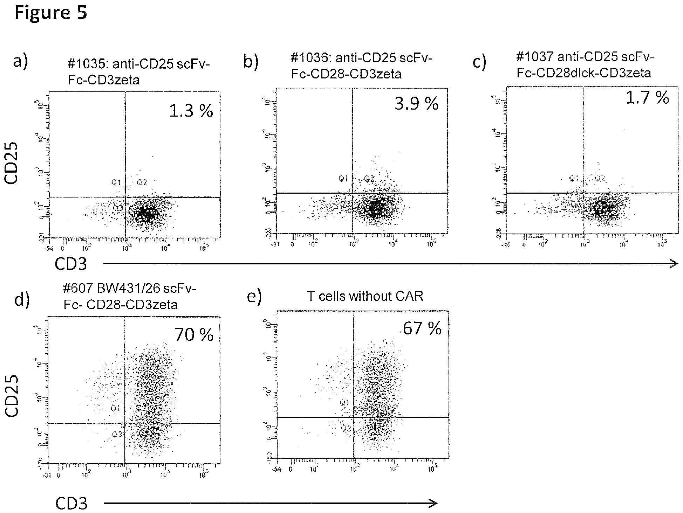

**Figure 5****.** *Surface expression of CD25 on CAR-modified T cells.*
   Human T cells were retrovirally transduced with the expression constructs for the anti-CD25 CAR #1035 (a), anti-CD25 CAR #1036 (b) and anti-CD25 CAR #1037 (c), respectively (10⁶ cells each). T cells transduced with the anti-CEA receptor BW431/26scFv-Fe-CD28-CD3 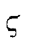 #607 and T cells without CAR served as controls (d and e). After 48 hours, CD25 expression by CD3⁺ T cells was detected by flow cytometry using a PE-conjugated anti-CD25 antibody and a FITC conjugated anti-CD3 antibody. CD3⁺ T cells with CD25 expression are substantially reduced in cultures with anti-CD25 CAR T cells compared with cultures with anti-CEA CAR T cells or T cells without CAR.

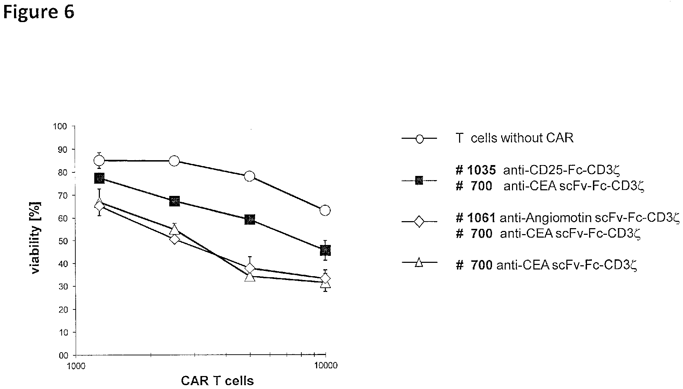

**Figure 6****.** *Anti-CD25 CAR T cells reduce the cytotoxic activity of T cells with anti-CEA CAR.*
   T cells from the peripheral blood of the same donor were engineered with the anti-CEA CAR BW431/26-Fc-CD3 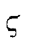 #700, the anti-CD25 CAR #1035 and the anti-angiomotin CAR #1061, respectively. Increasing numbers of these T cells (0.125 - 1x10⁴ cells per well) were incubated with CEA+ cells (10⁴ cells per well) of the LS174T line. After 48 hours, the number of living cells was determined by an XTT-based viability test; data represent the mean ± standard error of the mean.
   T cells with the anti-CEA CAR BW431/26-Fc-CD3 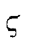 #700 reduce the viability of CEA+ cells of the LS174T line. When these anti-CEA CAR T cells were co-incubated with T cells with the anti-CD25 CAR #1035, the cytolytic activity against CEA+ LS174T cells was reduced. For comparison, T cells expressing the anti-angiomotin CAR #1061 did not have the effect on the anti-CEA CAR #700 T cells. T cells without CAR served as further controls.

### EXAMPLES

### EXAMPLE 1

### Materials and Methods

### 1. Cell lines and reagents.

The colon carcinoma cell line LS174T (ATCC CL-188) was obtained from ATCC, Rockville, MD, USA. Anti-CD3 mAb OKT3 and anti-CD28 mAb 15E8 were purified from OKT3 hybridoma (ATCC CRL 8001) and 15E8 hybridoma (kindly provided by Dr. R. van Lier, Red Cross Central Blood Bank, Amsterdam, The Netherlands) supernatants, respectively, by affinity chromatography.

Matched antibody pairs for capture and detection of human IFN-γ were purchased from BD Biosciences. Recombinant IL-2 was obtained from Endogen, Woburn, MA, USA. Immunofluorescence was analyzed using a FACS-Canto™ cytofluorometer equipped with the Diva software (Becton Dickinson, Mountain View, CA, USA).

### 2. Preparation of human T cells.

Peripheral blood lymphocytes were obtained from healthy donors by Ficoll density centrifugation. T cells were activated initially by incubation with the agonistic anti-CD3 antibody OKT3 and anti-CD28 antibody 15E8 (100 ng/ml each) and further cultivated in the presence of IL-2 (500 U/ml).

### 3. CARs.

Engineering of CARs with specificity for the carcinoembryonic antigen (CEA) and the retroviral modification of T cells was previously described in detail by Hombach *et al.* (2010), Weijtens *et al.* (1998), Hombach *et al.* (2001) and Hombach *et al.* (2000).

The generation of anti-CD25 scFv was described by Barth *et al.* (1998).

### 4. T cell modification.

Human peripheral blood T cells were retrovirally transduced for CAR expression (Golumba-Ngy *et al.,* 2017). T cells were stimulated with OKT3 and 15E8 antibodies and transduced on day 2 or 3 by γ-retrovirus containing supernatants or by co-culture with virus producing 293T cells as described by Hombach *et al.* (2016). Retroviruses were produced by 293T cells upon transient transfection with the DNA of the GALV encoding and the gag/pol encoding helper plasmids, and the plasmid encoding the respective CAR. CAR expression was monitored by flow cytometry using an antibody against the common extracellular IgG1 Fc domain.

CAR expression was monitored by flow cytometry using an antibody against the common extracellular IgG1 Fc domain.

### 5. Flow cytometry and cell sorting.

For flow cytometric analysis and cell sorting CAR engineered T cells were stained with fluorochrome-labeled antibodies specific for IgG1(to detect the CAR) and CD3, respectively, and recorded by a FACSCanto II flow cytometer equipped with the FACSDiva software (BD Bioscience). CD4⁺ and CD8⁺ CAR T cells were purified by flow sorting using a FACSAria III cell sorter (BD Bioscience). Doublets were discriminated using FSC-A versus FSC-W and SSC-A versus SSC-W gating.

### 6. Statistics.

Experimental results from independent representative experiments are reported as mean values + standard deviation (SD). Significance analyzes were performed by the two-sided Student's t test using Microsoft Excel and Graphpad Prism, respectively.

### EXAMPLE 2

### Expression of the anti-CD25 CARs in human T cells

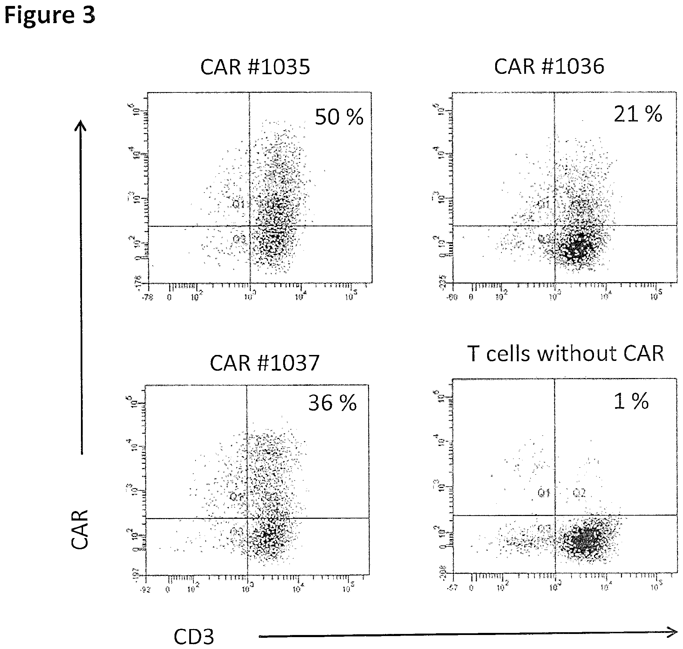

T cells were engineered *in vitro* with the anti-CD25 CAR #1035, #1036, #1037, respectively, by retroviral transduction. The CAR on the T cell surface was recorded by flow cytometry using an anti-human IgG1 antibody that recognizes the common extracellular IgG1 Fc spacer domain. T cells were recorded by staining for CD3. Transduced T cells express the CAR on the cell surface (Figure 3). Non-transduced T cells do not express a CAR.

### EXAMPLE 3

### Specific activation of CAR T cells by antibody-mediated CAR crosslinking

CAR T cells were assayed for CAR redirected function by antibody mediated crosslinking the CAR. Therefore, 10⁴ CAR T cells were incubated on microtiter plates coated with an anti-human IgG1 antibody that recognizes the common extracellular CAR spacer. As controls the plates were coated with a mouse IgG antibody of irrelevant specificity, with the agonistic anti-CD3 antibody OKT3, and with both the OKT3 antibody and the anti-CD28 antibody 15E8, respectively. T cells without CAR or with the anti-CEA CAR BW431/26-Fc-CD28-CD3 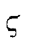 # 607 served for comparison. After 48 hrs the culture supernatant was recorded for the pro-inflammatory cytokines IFN-γ and IL-2 by ELISA (Figure 4).

Crosslinking the CAR by the immobilized anti-human IgG1 antibody induced the release of IFN-γ indicating T cell activation. Activation was specifically induced by the CAR since T cells without CAR did not increase IFN-γ release. IFN-γ levels were higher upon CD28-ζ CAR #1036 stimulation compared with stimulation of the ζ CAR #1035 without CD28 costimulation. For comparison T cell activation was also obtained independently of the CAR upon TCR/CD28 stimulation after OKT3 plus 15E8 antibody incubation but less upon incubation with only OKT3.

T cells with the anti-CD25scFv-Fc-CD28-CD3ζ CAR #1036 released in addition IL-2 which was also the case for T cells with the anti-CEA CAR BW431/26scFv-Fc-CD28-CD3 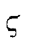 # 607. T cells with the anti-CD25 CAR without the CD28 costimulatory domain (#1035) did release only low levels of IL-2 as did T cells with the anti-CD25 CAR 1037 with CD28Δlck domain. All T cells used in the assay can equally be activated since CD3/CD28 stimulation by the antibodies OKT3 plus 15E8 produced IFN-γ release at similar levels.

### EXAMPLE 4

### Anti-CD25 CAR T cells reduce the number of CD25+ T cells in vitro

CD25 is highly expressed by activated T cells and regulatory T cells. We asked whether cytolytic T cells engineered with an anti-CD25 CAR reduce the number of CD25+ T cells *in vitro.*

T cells from the peripheral blood were engineered with the anti-CD25 CAR #1035, #1036, and #1037, respectively. T cells engineering with the CEA-specific CAR #700 and T cells without CAR served as controls. Expression of the respective CAR was confirmed by flow cytometry. CAR T cells were stimulated for 48 hrs with IL-2 plus the agonistic anti-CD3 antibody OKT3 and afterwards incubated without stimulation for 12 hrs until recording the number of CD25+ cells by flow cytometry.

The number of CD25+ T cells was reduced in anti-CD25 CAR T cell cultures compared with cultures that contain T cells with the anti-CEA CAR or T cells without a CAR (Figure 5). Reduction of CD25+ T cell numbers was observed in all cultures with anti-CD25 CAR T cells indicating that each anti-CD25 CAR is capable to redirect T cells for reducing the number of CD25+ T cells.

### EXAMPLE 5

### Modulating the cytotoxicity of anti-CEA CAR T cells by anti-CD25 CAR T cells.

Anti-CEA CAR T cells produce a pro-inflammatory reaction by releasing cytokines and eliminating CEA+ cells. Anti-CD25 CAR T cells were used to repress the pro-inflammatory reaction of anti-CEA CAR T cells that serve as model for any inflammatory immune response.

Peripheral blood T cells were *in vitro* engineered with the anti-CEA CAR BW431/26scFv-Fc-CD3 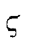 #700 to be used as pro-inflammatory cells redirected against CEA+ target cells. A second population of T cells from the same donor was engineered *in vitro* with the anti-CD25scFv-Fc-CD3 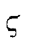 CAR #1035 to serve as anti-inflammatory T cells. As control T cells were engineered with the angiomotin specific CAR #1061 of irrelevant specificity.

Anti-CD25 CAR #1035 T cells in increasing numbers (0.125 - 1x10⁴ cells per well) were co-incubated with CEA+ LS174T cells (10⁴ cells per well) and anti-CEA CAR #700 T cells (0.125 - 1x10⁴ cells per well). After 48 hrs the viability of LS174T cells was determined. Anti-CEA CAR T cells eliminate CEA+ LS174T cells dependent on the number of anti-CEA CAR T cells (Figure 6). In the presence of anti-CD25 CAR T cells, the lytic activity of anti-CEA CAR T cells was reduced indicated by higher viability of LS174T cells. Reduction of the cytolytic reaction by anti-CD25 CAR T cells was specific since anti-angiomotin CAR T cells did not alter the cytolytic activity of anti-CEA CAR T cells. Data indicate that anti-CD25 CAR T cells have the capacity to reduce the inflammatory capacity of an antigen-specific immune response.

### REFERENCES

Barth S, Huhn M, Wels W, Diehl V, Engert A. Construction and in vitro evaluation of RFT5(scFv)-ETA', a new recombinant single-chain immunotoxin with specific cytotoxicity toward CD25+ Hodgkin-derived cell lines. Int J Mol Med 1998;1:249-56.
Golumba-Nagy V, Kuehle J, Abken H. Genetic Modification of T Cells with Chimeric Antigen Receptors: A Laboratory Manual. Hum Gene Ther Methods 2017;28:302-9. doi:10.1089/hgtb.2017.083.
Hombach A, Heuser C, Gerken M, Fischer B, Lewalter K, Diehl V, et al. T cell activation by recombinant FcepsilonRI gamma-chain immune receptors: an extracellular spacer domain impairs antigen-dependent T cell activation but not antigen recognition. Gene Ther 2000;7:1067-75. doi:10.1038/sj.gt.3301195.
Hombach A, Wieczarkowiecz A, Marquardt T, Heuser C, Usai L, Pohl C, et al. Tumor-specific T cell activation by recombinant immunoreceptors: CD3 zeta signaling and CD28 costimulation are simultaneously required for efficient IL-2 secretion and can be integrated into one combined CD28/CD3 zeta signaling receptor molecule. J Immunol Baltim Md 1950 2001;167:6123-31.
Hombach A, Hombach AA, Abken H. Adoptive immunotherapy with genetically engineered T cells: modification of the IgG1 Fc "spacer" domain in the extracellular moiety of chimeric antigen receptors avoids "off-target" activation and unintended initiation of an innate immune response. Gene Ther 2010;17:1206-13. doi:10.1038/gt.2010.91.
Hombach AA, Görgens A, Chmielewski M, Murke F, Kimpel J, Giebel B, et al. Superior Therapeutic Index in Lymphoma Therapy: CD30(+) CD34(+) Hematopoietic Stem Cells Resist a Chimeric Antigen Receptor T-cell Attack. Mol Ther J Am Soc Gene Ther 2016;24:1423-34. doi:10.1038/mt.2016.82.
Weijtens ME, Willemsen RA, Hart EH, Bolhuis RL. A retroviral vector system "STITCH" in combination with an optimized single chain antibody chimeric receptor gene structure allows efficient gene transduction and expression in human T lymphocytes. Gene Ther 1998;5:1195-203. doi:10.1038/sj.gt.3300696.

## Claims

1. A protein comprising
(i) a CD25-specific binding domain, preferably comprising an anti-CD25 single chain Fv (scFv) fragment;
(ii) a linker domain, connecting domain (i) and domain (iii), preferably comprising a human immunoglobulin Fc domain, more preferably comprising human IgG1 Fc;
(iii) a transmembrane domain, and
(iv) a signalling domain,
wherein the signalling domain comprises
a primary human signal chain,
preferably derived from human CD3 zeta chain or human FcεRI gamma chain,
intracellular co-stimulatory signalling chain(s),
preferably derived from human CD28, 4-1BB, OX40 or CD27, or
a fusion of said intracellular co-stimulatory signal chain(s) with the intracellular domain of a primary human signal chain.

2. The protein of claim 1, wherein the signalling domain (iv) is derived from
(1) the human CD3 zeta chain,
(2) the intracellular domain of human CD28 linked to the intracellular domain of human CD3 zeta chain, and
(3) the intracellular domain of human CD28 linked to the intracellular domain of human CD3 zeta chain, wherein CD28 comprises at least one mutation, preferably a deletion of the Lck binding site.

3. The protein of claim 1 or 2, wherein the extracellular CD25-specific binding domain (i) is an anti-CD25 single chain Fv (scFv) fragment,
preferably comprising the amino acid sequence of SEQ ID NO.1,
or wherein the extracellular CD25-specific binding domain (i) is derived from or comprises IL-2.

4. The protein of any one of claims 1 to 3, wherein the linker domain (ii) is human IgG1 Fc domain or a domain derived therefrom,
preferably comprising the amino acid sequence of SEQ ID NOs. 2 or 3.

5. The protein of any one of claims 1 to 4, further comprising an N-terminal secretion signal peptide, preferably immunoglobulin heavy chain signal peptide, such as the IgG kappa light chain leader sequence.

6. The protein of any one of claims 1 to 5, comprising an amino acid sequence selected from SEQ ID NOs. 4 to 6,
or an amino acid sequence that has at least 85%, preferably 90% or 95 % sequence identity to an amino acid sequence of SEQ ID NOs. 4 to 6.

7. A nucleic acid encoding the protein of any one of claims 1 to 6.

8. The nucleic acid of claim 7, comprising the nucleic acid encoding for the amino acid sequence of SEQ ID NO. 1 or
comprising the nucleic acid sequence of SEQ ID NO. 7 or their complementary sequences or sequences that have at least 85%, preferably 90% or 95 % sequence identity,
and/or comprising the nucleic acid encoding for the amino acid sequence of SEQ ID NOs. 2 or 3,
comprising the nucleic acid sequence of SEQ ID NOs. 8 or 9, or their complementary sequences or sequences that have at least 85%, preferably 90% or 95 % sequence identity.

9. The nucleic acid of claim 7 or 8, comprising the nucleic acid encoding for an amino acid sequence selected from SEQ ID NOs. 4 to 6 or a nucleic acid sequence selected from SEQ ID NOs. 10 to 12 or their complementary sequences or sequences that have at least 85%, preferably 90% or 95 % sequence identity.

10. An expression construct for expressing the protein of any one of claims 1 to 6 in a cell.

11. A host cell expressing a protein of any one of claims 1 to 6 or comprising a nucleic acid of any of claims 7 to 9 or an expression construct of claim 10,
which is preferably selected from cells of the immune system, more preferably T cells or regulatory T (Treg) cells.

12. Use of a protein of claims 1 to 6, a nucleic acid of claims 7 to 9 or an expression construct of claim 10 for generating CD25-specific immune cells, preferably CD25-specific T cells or CD25-specific regulatory T (Treg) cells.

13. A pharmaceutical composition, comprising
(i) at least one protein of claims 1 to 6, at least one nucleic acid of claims 7 to 9, at least one expression construct of claim 10 or at least one host cell of claim 11, and
(ii) optionally, pharmaceutically acceptable excipient(s) and/or carrier, preferably harbouring (i).

14. The protein of claims 1 to 6, the nucleic acid of claims 7 to 9, the expression construct of claim 10 or the host cell of claim 11 for use as a medicament.

15. The protein of claims 1 to 6, the nucleic acid of claims 7 to 9, the expression construct of claim 10 or the host cell of claim 11 for use in the treatment of inflammation,
wherein inflammation comprises chronic inflammation, in particular in autoimmune diseases, such as multiple sclerosis, inflammatory bowel disease including Crohn's disease and ulcerative colitis, type-1 diabetes, psoriasis, rheumatoid arthritis, systemic lupus erytematosus, Hashimoto's thyreoiditis, Addison's disease, Graves' disease, Sjögren's syndrome, myastenia gravis, auto-immune vasculitis, pernicious anemia, and celiac disease,
preferably comprising reducing or supressing the inflammatory reaction in tissues,
or the protein of claims 1 to 6, the nucleic acid of claims 7 to 9, the expression construct of claim 10 or the host cell of claim 11 for use in target-cell specific immunotherapy.
